# EUROPÄISCHE PATENTSCHRIFT

(11) **EP 0 867 444 B1**
(45) Veröffentlichungstag und Bekanntmachung des Hinweises auf die Patenterteilung: **08.10.2003**
(21) Anmeldenummer: 98250096.9
(22) Anmeldetag: 20.03.1998
(51) Int. Cl.: C07F 7/18, C08G 77/58, A61K 6/093, C08G 77/22

(54) **Hydrolysierbare und polymerisierbare Vinylcyclopropansilane**
Hydrolyzable and polymeryzable vinylcyclopropanesilanes
Vinylcyclopropanesilanes hydrolysables et polymérisables

(30) Priorität: 25.03.1997 DE 19714320
(43) Veröffentlichungstag der Anmeldung: 30.09.1998
(73) Patentinhaber: Ivoclar Vivadent AG, 9494 Schaan (LI)
(72) Erfinder: Moszner, Norbert, Prof. Dr., 9420 Eschen (LI); Völkel, Thomas, Dr., 88131 Lindau (DE); Zeuner, Frank, Dr., 9490 Vaduz (LI); Stein, Sabine, 6710 Nenzing (AT); Rheinberger, Volker, Dr., 9490 Vaduz (LI)
(74) Vertreter: UEXKÜLL & STOLBERG

(56) Entgegenhaltungen:
- EP-A- 0 058 514

## Beschreibung

Die Erfindung betrifft hydrolysierbare und polymerisierbare Vinylcyclopropansilane, Verfahren zu ihrer Herstellung, aus ihnen hergestellte Kieselsäurekondensate, Polymerisate und Zusammensetzungen sowie die Verwendung all dieser Materialien unter anderem zur Herstellung von makromolekularen Massen und zur Herstellung von Verbundmaterialien, Adhäsiven, Beschichtungen und insbesondere von Dentalmaterialien.

Hydrolysierbare Silane, die polymerisierbare organische Reste enthalten, finden bei der Herstellung von Beschichtungen, partikulären Füllstoffen, Klebemassen und monolithischen Formkörpern sowie bei der Oberflächenmodifizierung von Verstärkungsstoffen Anwendung. Dabei werden die Silane alleine, in Mischung mit anderen Silanen oder in Gegenwart von anderen Metallalkoxiden hydrolytisch kondensiert und thermisch, photochemisch oder redoxinitiiert polymerisiert, d.h. gehärtet.

Im Zusammenhang mit der Herstellung von organisch-anorganischen Verbundmaterialien sind vor allem organisch-modifizierte Silane mit polymerisationsfähigen organischen Gruppen, wie Vinyl-, (Meth)acryl-, Allyl- oder Styryl-Gruppen von besonderem Interesse, da sie den simultanen oder konsekutiven Aufbau sowohl eines anorganischen als auch eines organischen Netzwerkes und damit von Verbundmaterialien mit maßgeschneiderten Eigenschaften gestatten (vgl. H. Schmidt, Mat. Res. Soc. Symp. Proc. Vol. 32 (1984), 327-335; H. Schmidt, H. Wolter, J. Non-Cryst. Solids 121 (1990), 428-435). Dabei werden die polymerisationsfähigen Silane in der Regel zunächst in Lösung hydrolytisch kondensiert. Nach Zugabe von thermischem Initiator oder Photoinitiator und Abtrennung des Lösungsmittels bilden sich dann nanopartikuläre Harze, die nach Formgebung polymerisiert und damit gehärtet werden.

Ein wesentlicher Nachteil dieser Materialien besteht jedoch darin, daß die bei der Polymerisation erfolgende Ausbildung des organischen Netzwerkes meist von einer beträchtlichen Volumenkontraktion begleitet ist, die zur Deformation der Formkörper, zur Verminderung der Substrathaftung, zur Schichtentrennung, zur Ausbildung von Hohlräumen oder zur Ausbildung von Materialspannungen führen kann. Eine verringerte Volumenkontraktion erfolgt bei Silanen, die ringöffnende Gruppen tragen. In diesem Zusammenhang beschreiben die EP-B-0 358 011 kratzfeste Materialien u.a. auf der Basis von 3-Glycidyloxypropylsilanen, die EP-B-0 486 469 organisch-anorganische Hybridpolymere von 3-Glycidyloxypropylsilanen und die DE-C-41 33 494 Dentalharzmassen, bei denen z.B. Silane mit ringöffnenden Spiroorthoestergruppen eingesetzt werden. Dabei erweist es sich allerdings als nachteilig, daß bei Silanen mit Epoxid- oder Spiroorthoester-Gruppen nur kationisch eine Ringöffnung möglich ist und diese Silane damit nur in Abwesenheit von Feuchtigkeit polymerisieren, ferner die Polymerisation der Epoxidsilane erst bei erhöhten Temperaturen hinreichend schnell abläuft und die Spiroorthoestersilane eine nur geringe Stabilität zeigen.

Weiter sind als siliciumhaltige Vinylcylopropan-Derivate nur trimethylsiloxy-substituierte Vinylcyclopropane (vgl. J. Amer. Chem. Soc. 116 (1994), 6453-6454) und 1-Trialkylsilyl-2-vinylcyclopropane (M. Katsukiyo et al. Tetrahedron Lett. 30 (1989), 4413-4416) bisher bekannt geworden, d.h. Verbindungen der folgenden Formel:

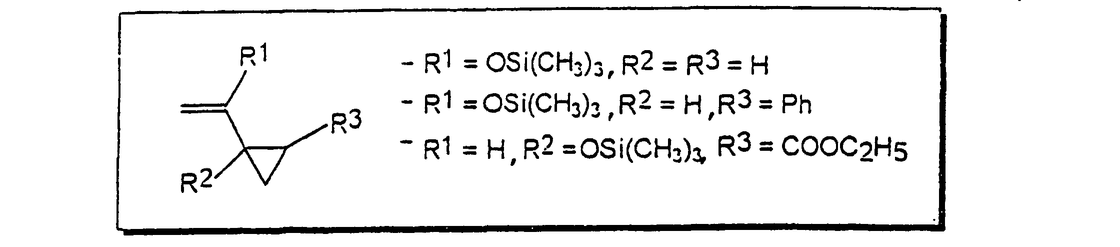

Die EP 058 514 betrifft strahlungshärtbare Silan-Adhäsive, die aus einem polymerisierbaren Harz und mindestens 20% eines polymerisierbaren Silans bestehen. Dabei ist das polymerisierbare Silan vorzugsweise ein Acryl- oder Vinyl-Silan. Die beschriebenen Adhäsive lassen sich für Füllungen von dentalen Kavitäten einsetzen.

Der Erfindung liegt nunmehr die Aufgabe zugrunde, hydrolysierbare und polymerisierbare Vinylcyclopropansilane zur Verfügung zu stellen, aus denen allein oder zusammen mit anderen hydrolytisch kondensierbaren und polymerisierbaren Komponenten Zusammensetzungen herstellbar sind, die nur unter geringem Schrumpf polymerisieren und als Verbund- oder Beschichtungsmaterial, Klebstoff, Haftvermittler oder zur Herstellung von Füllstoffen oder Materialien für medizinische oder dentale Zwecke geeignet sind. Diese Silane sollen sich in organisch-anorganische Verbundmaterialien kovalent einbauen lassen und synthetisch so zugänglich sein, daß der Abstand zwischen Silicium und den polymerisierbaren Gruppen variiert werden kann.

Diese Aufgabe wird erfindungsgemäß durch die hydrolysierbaren und polymerisierbaren Vinylcyclopropansilane nach den Ansprüchen 1 bis 3 gelöst. Die Erfindung betrifft weiter die Kieselsäurekondensate nach Anspruch 4, die Polymerisate nach Anspruch 5, die Zusammensetzungen nach den Ansprüchen 6 und 7 sowie die Verwendung nach Anspruch 8.

Die erfindungsgemäßen hydrolysierbaren und polymerisierbaren Vinylcyclopropansilane und deren Stereoisomere entsprechen der allgemeinen Formel (I) : 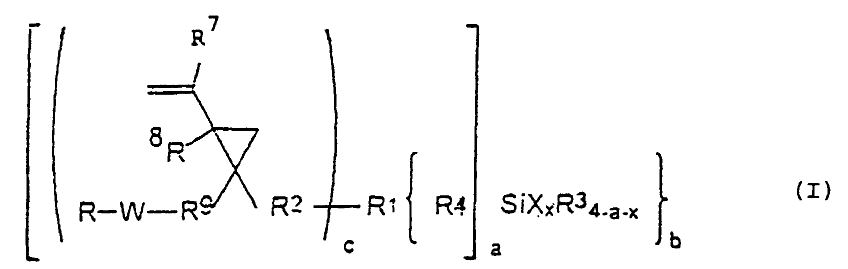 wobei die Variablen R, R¹, R², R³, R⁴, R⁵, R⁶, R⁷, R⁸, R⁹, W, X, Y, a, b, c und x unabhängig voneinander die folgenden Bedeutungen haben:
- R =: Wasserstoff, substituiertes oder unsubstituiertes C₁- bis C₁₂-Alkyl, C₇- bis C₁₅-Alkylaryl oder C₆- bis C₁₄-Aryl, oder R³₃₋ₓXₓSi-R₄-R₁-R₂-;
- R¹ =: entfällt oder substituiertes oder unsubstituiertes C₁- bis C₁₈-Alkylen, C₆- bis C₁₈-Arylen, C₇- bis C₁₈-Alkylenarylen oder -Arylenalkylen, wobei diese Reste durch mindestens eine Gruppe ausgewählt aus Ether-, Thioether-, Ester-, Carbonyl-, Amid- und Urethangruppe unterbrochen sein oder diese endständig tragen können;
- R² =: entfällt oder substituiertes oder unsubstituiertes C₁- bis C₁₈-Alkylen, C₆- bis C₁₈-Arylen, C₇- bis C₁₈-Alkylenarylen oder C₇- bis C₁₈- Arylenalkylen, wobei diese Reste durch mindestens eine Gruppe ausgewählt aus Ether-, Thioether-, Ester-, Thioester-, Carbonyl-, Amid- und Urethangruppe unterbrochen sein und/oder diese endständig tragen können;
- R³ =: entfällt oder substituiertes oder unsubstituiertes C₁- bis C₁₈-Alkyl, C₂- bis C₁₈-Alkenyl, C₆- bis C₁₈-Aryl, C₇- bis C₁₈-Alkylaryl oder C₇- bis C₁₈-Arylal-kyl, wobei diese Reste durch mindestens eine Gruppe ausgewählt aus Ether-, Thioether-, Ester-, Carbonyl-, Amid- und Urethangruppe unterbrochen sein können;
- R⁴ =: entfällt oder substituiertes oder unsubstituiertes -CHR⁶-CHR⁶-, -CHR⁶-CHR⁶-S-R⁵-, -S-R⁵-, -Y-CO-NH-R⁵- oder -CO-O-R⁵-;
- R⁵ =: substituiertes oder unsubstituiertes C₁- bis C₁₈-Alkylen, C6- bis C₁₈-Arylen, C₆- bis C₁₈-Alkylenarylen oder C₆- bis C₁₈-Arylenalkylen, wobei diese Reste durch mindestens eine Gruppe ausgewählt aus Ether-, Thioether-, Ester-, Carbonyl-, Amid- und Urethangruppe unterbrochen sein können;
- R⁶ =: Wasserstoff oder substituiertes oder unsubstituiertes C₁- bis C₁₈-Alkyl oder C₆ bis C₁₀-Aryl;
- R⁷ =: Wasserstoff oder substituiertes oder unsubstituiertes C₁- bis C₁₀-Alkyl, oder Halogen oder Hydroxy;
- R⁸ =: Wasserstoff oder substituiertes oder unsubstituiertes C₁- bis C₁₀-Alkyl;
- R⁹ =: entfällt oder substituiertes oder unsubstituiertes C₁- bis C₁₀-Alkylen;
- W =: entfällt oder Carbonyl-, Ester-, Ether-, Thioether-, Amid- oder Urethangruppe;
- X =: eine hydrolysierbare Gruppe, nämlich Halogen, Hydroxy, Alkoxy oder Acyloxy;
- Y =: O oder S;
- a =: 1, 2 oder 3;
- b =: 1, 2 oder 3;
- c =: 1 bis 6; und
- x =: 1, 2 oder 3;
und mit der Maßgabe, daß
(i) a+x = 2, 3 oder 4
   und
(ii) a und/oder b = 1.

Die obige Formel decken jedoch nur solche Verbindungen ab, die mit der Valenzlehre zu vereinbaren sind.

Üblicherweise liegen die erfindungsgemäßen Silane als Stereoisomeren-Gemische und insbesondere als Racemate vor.

Die bei den Resten möglicherweise vorhandenen Ether-, Thioether-, Ester-, Thioester-, Carbonyl-, Amid- und Urethangruppen sind durch die folgenden Formeln definiert: -O-, -S-, -CO-O-, -O-CO-, -CO-S-, -S-CO-, -CS-O-, -O-CS-, -CO-, -CO-NH-, -NH-CO-, -O-CO-NHund -NH-CO-O-.

Die in den Formeln (I) möglichen nicht-aromatischen Reste oder nicht-aromatischen Teile der Reste können geradkettig, verzweigt oder cyclisch sein.

Alkylreste haben bevorzugt 1 bis 8 und besonders bevorzugt 1 bis 4 Kohlenstoffatome. Spezielle Beispiele für mögliche Alkylreste sind Methyl, Ethyl, n- und iso-Propyl, sec.- und tert.-Butyl, n-Pentyl, Cyclohexyl, 2-Ethylhexyl und Octadecyl.

Alkenylreste haben bevorzugt 2 bis 10 und besonders bevorzugt 2 bis 6 Kohlenstoffatome. Spezielle Beispiele für mögliche Alkenylreste sind Vinyl, Allyl- und iso-Butenyl.

Bevorzugte Beispiel für mögliche Aryl-Reste sind Phenyl, Biphenyl und Naphthyl.

Alkoxyreste haben bevorzugt 1 bis 6 Kohlenstoffatome. Spezielle Beispiel für mögliche Alkoxyreste sind Methoxy, Ethoxy, n-Propoxy, iso-Propoxy und tert.-Butoxy.

Acyloxyreste haben vorzugsweise 2 bis 5 Kohlenstoffatome. Spezielle Beispiele sind Acetyloxy und Propionyloxy.

Bevorzugte Alkylenreste leiten sich von den obigen bevorzugten Alkylresten ab und bevorzugte Arylenreste leiten sich von den obigen bevorzugten Arylresten ab.

Bevorzugte aus einer Kombination von nicht-aromatischem und aromatischem Teil bestehende Reste, wie Alkylaryl-, Arylalkyl-, Alkylenarylen- und Arylenalkylenreste, leiten sich von den obigen bevorzugten Alkyl- und Arylresten ab. Spezielle Beispiele hierfür sind Benzyl, 2-Phenylethyl und Tolyl.

Die genannten substituierten R-Reste tragen einen oder mehrere einfache Substituenten. Beispiele für diese Substituenten sind Methyl, Ethyl, Phenyl, Benzyl, Hydroxymethyl, Hydroxyethyl, Methoxy, Ethoxy, Chlor, Brom, Hydroxy, Mercapto, Isocyanato, Vinyloxy, Acryloxy, Methacryloxy, Allyl, Styryl, Epoxy, Carboxy, SO₃H, PO₃H₂ oder PO₄H₂.

Für a, b, c oder x ≥ 2 können die Reste X und W sowie die einzelnen R-Reste jeweils dieselbe oder eine unterschiedliche Bedeutung haben.

Außerdem existieren für die oben angegebenen variablen der Formel (I) bevorzugte Definitionen, die unabhängig voneinander gewählt werden können und wie folgt sind:
- R =: C₁- bis C₅-Alkyl, Benzyl oder Phenyl oder R³ ₃₋ₓXₓSi-R₄-R₁-R₂-;
- R¹ =: C₁- bis C₈-Alkylen, wobei diese Reste durch mindestens eine Gruppe ausgewählt aus Ether-, Thioether-, Ester- und Urethangruppe unterbrochen sein können;
- R² =: entfällt oder C₁- bis C₈-Alkylen, wobei diese Reste durch mindestens eine Gruppe ausgewählt aus Ether-, Thioether-, Ester-, Thioester, Carbonyl-, Amid- und Urethangruppe unterbrochen sein oder diese endständig tragen können;
- R³ =: entfällt oder Methyl, Ethyl oder Phenyl;
- R⁴ =: entfällt oder -CHR⁶-CHR⁶-, -S-R⁵-, -Y-CO-NH-R⁵- oder -CO-O-R⁵-;
- R⁵ =: C₁- bis C₈-Alkylen, wobei diese Reste durch mindestens eine Gruppe ausgewählt aus Ether-, Thioether-, Ester-, Carbonyl-, Amid- und Urethangruppe unterbrochen sein können;
- R⁶ =: Wasserstoff oder C₁- bis C₅-Alkyl;
- R⁷ =: Wasserstoff oder C₁- bis C₅-Alkyl;
- R⁸ =: Wasserstoff oder C₁- bis C₅-Alkyl;
- R⁹ =: entfällt oder C₁- bis C₃-Alkylen;
- W =: Ester-, Amid- oder Urethangruppe;
- X =: Methoxy, Ethoxy oder Chlor;
- Y =: O oder S;
- a =: 1;
- b =: 1;
- c =: 1 bis 6;
- x =: 2 oder 3; und/oder
- a+x =: 3.

Dabei können die einzelnen R-Reste wiederum einfache Substituenten tragen.

Bevorzugte Verbindungen sind demgemäß solche, bei denen mindestens eine der Variablen der Formel (I) die vorstehend beschriebene bevorzugte Definition aufweist.

Weiter sind solche Vinylcyclopropansilane der Formel (I) bevorzugt, bei denen die Indices a, b und/oder c den Wert 1 haben, und Beispiel hierfür sind die Silane gemäß den nachstehenden allgemeinen Formeln (II), (III), (IV) und (V).

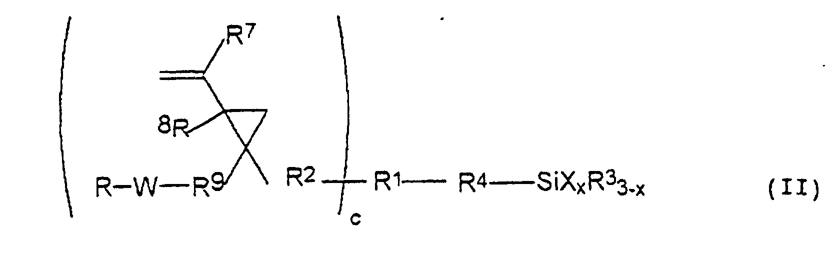

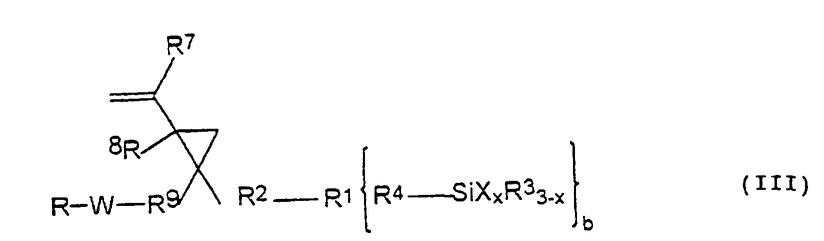

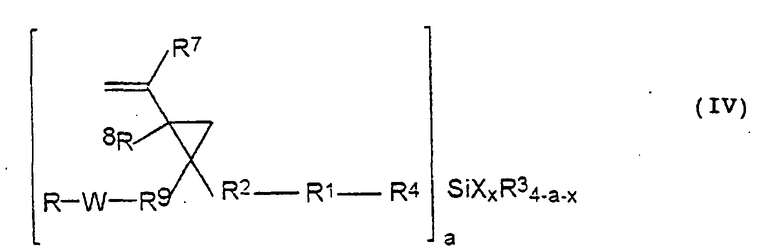

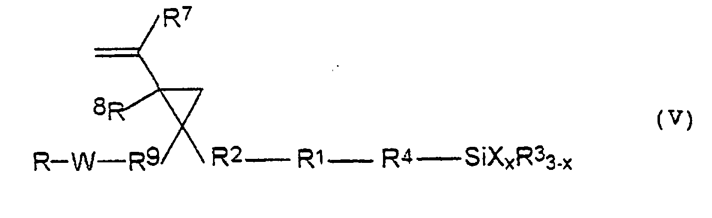

Spezielle Beispiele für bevorzugte erfindungsgemäße Vinylcyclopropansilane der Formel (I) sind im folgenden angegeben:

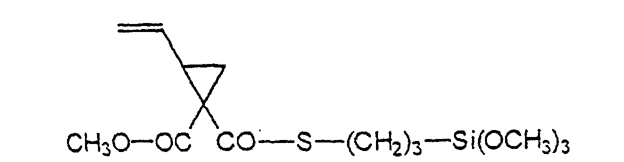

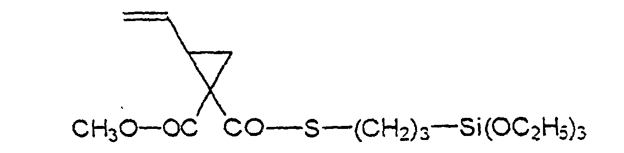

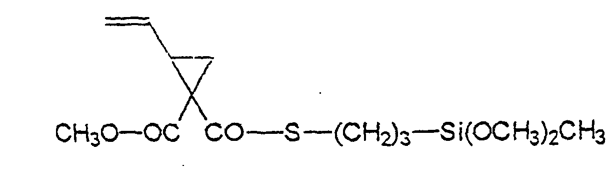

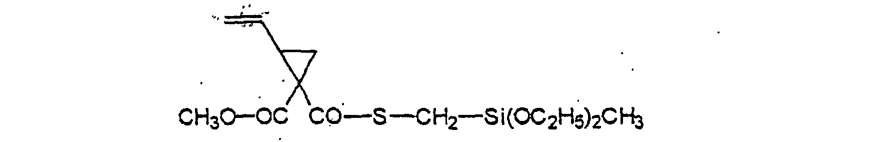

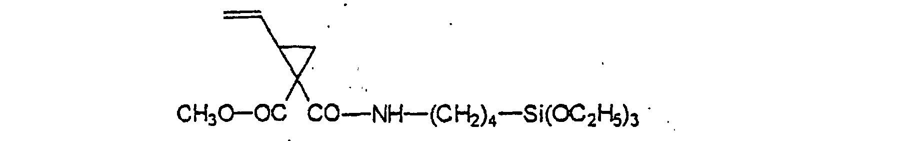

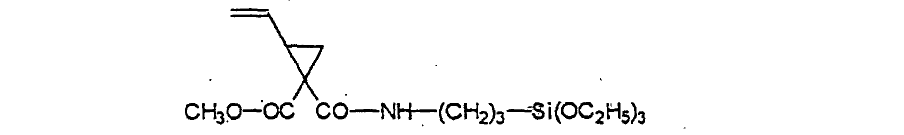

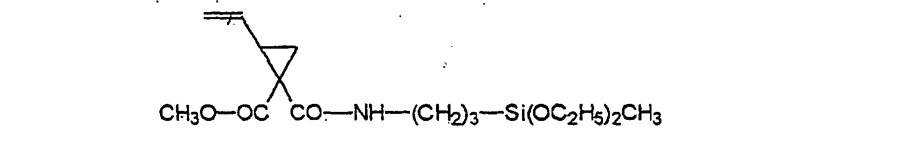

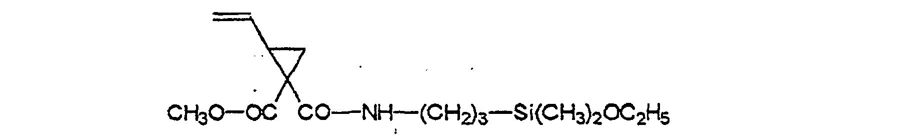

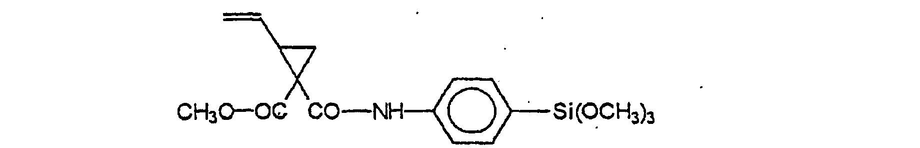

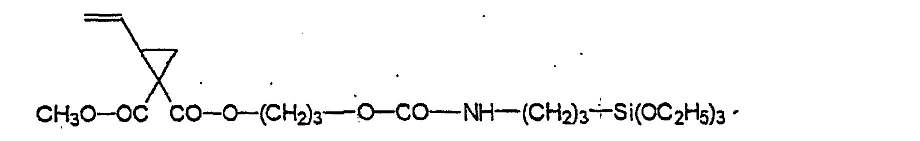

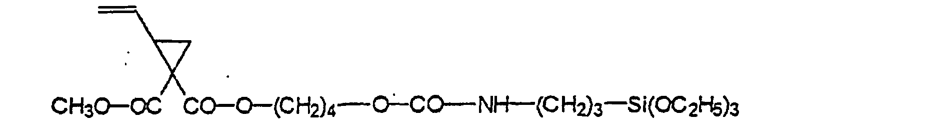

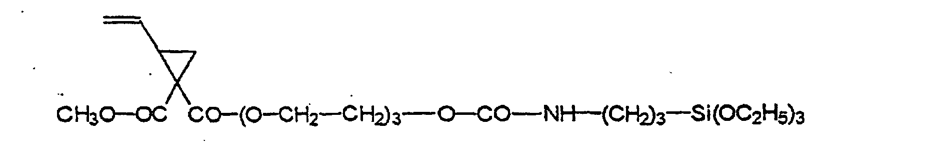

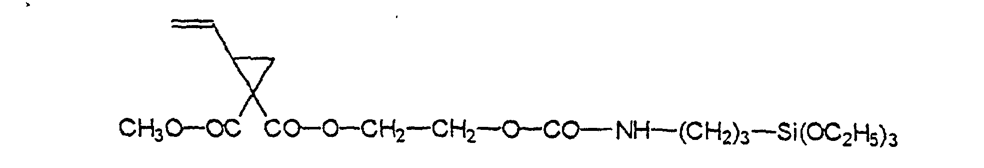

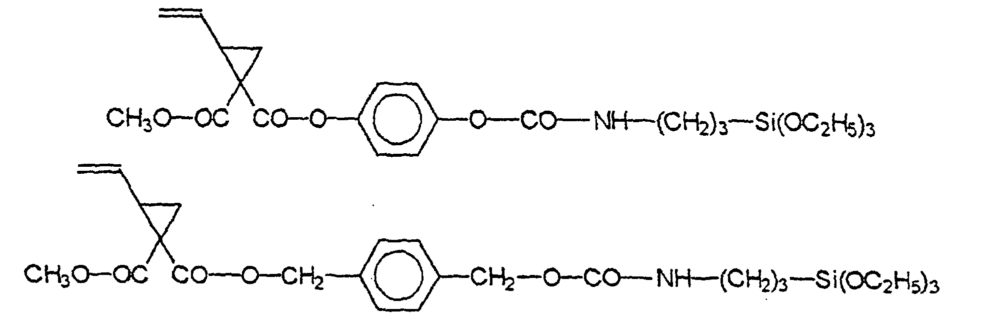

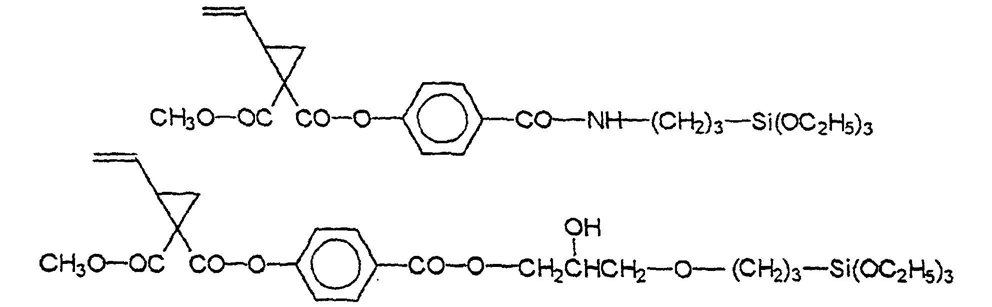

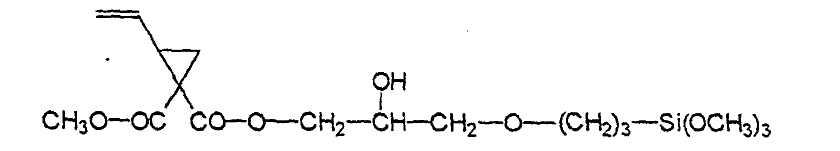

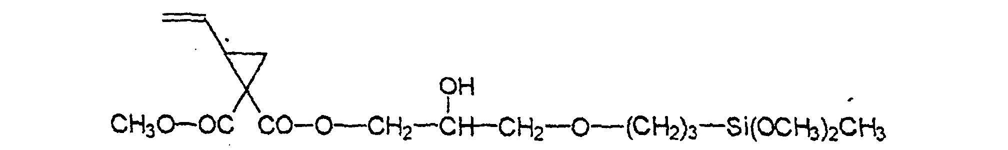

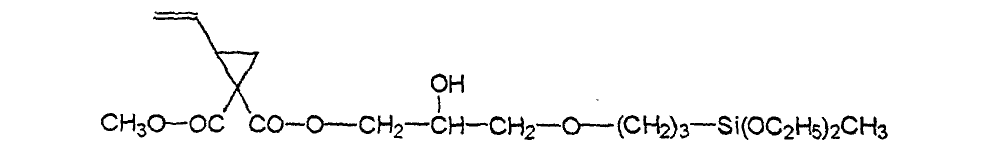

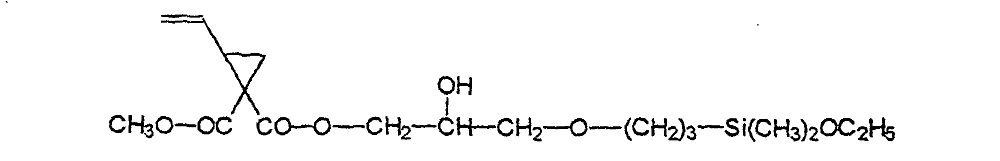

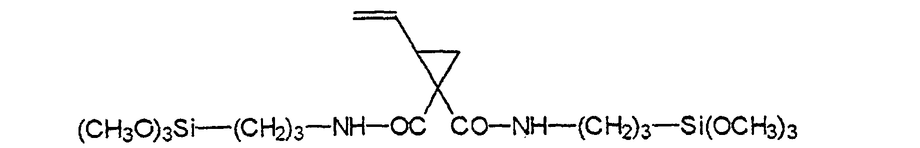

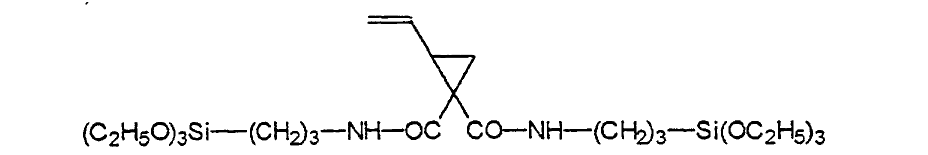

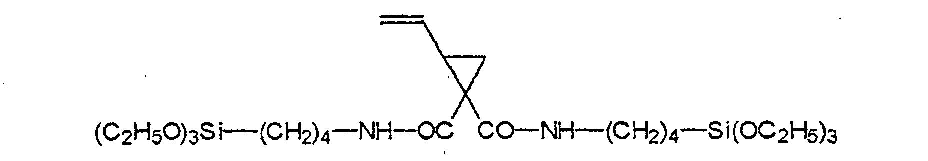

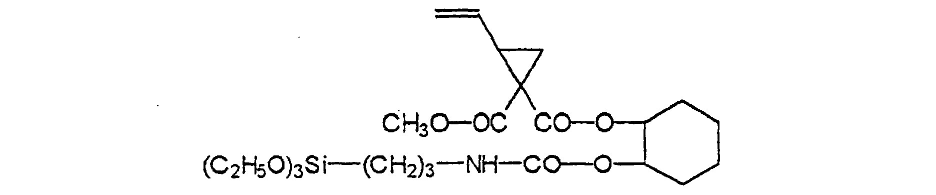

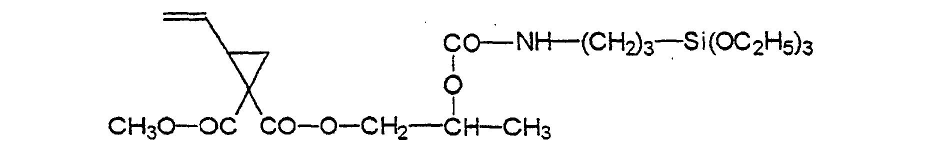

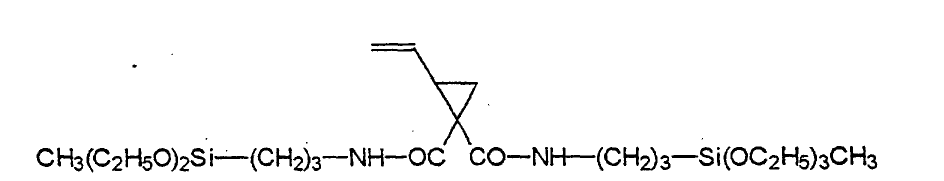

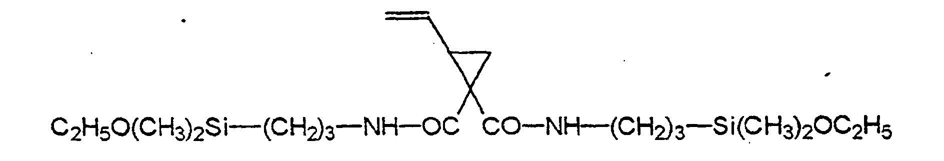

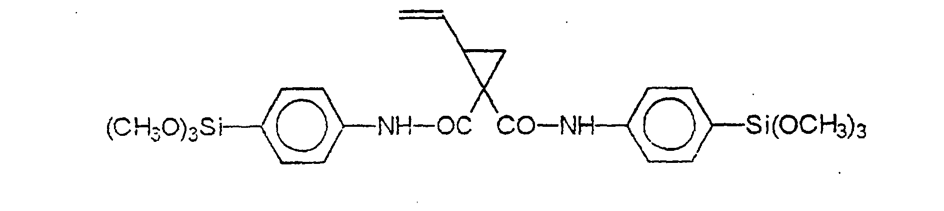

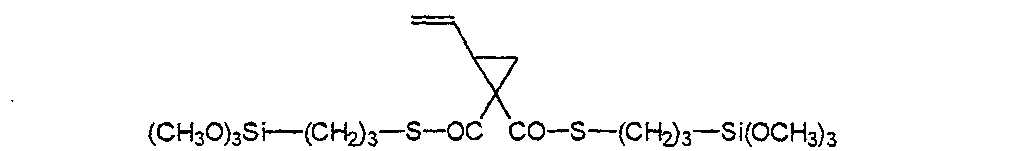

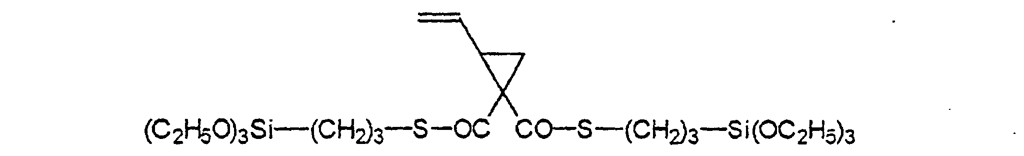

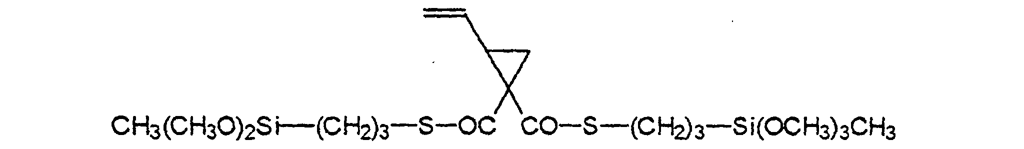

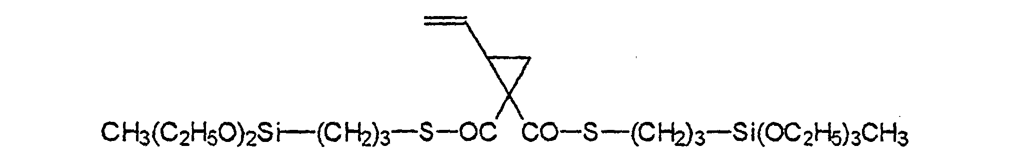

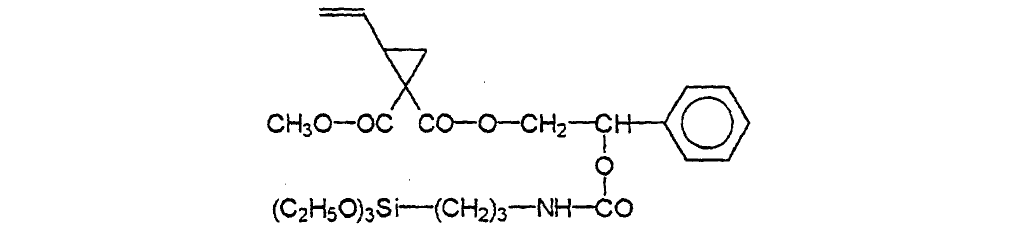

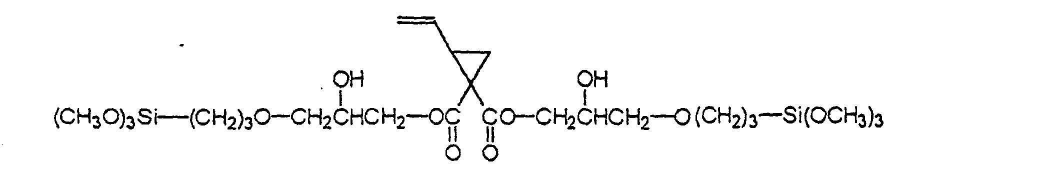

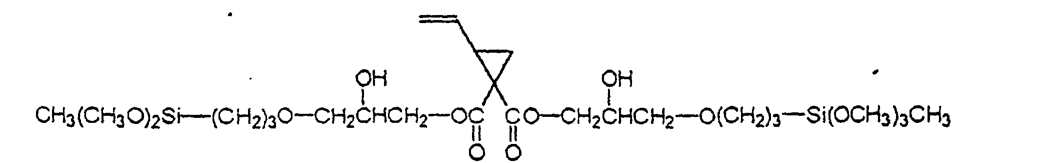

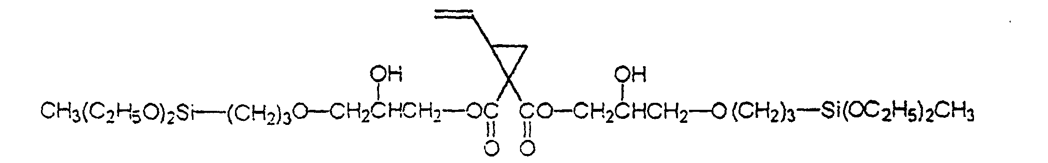

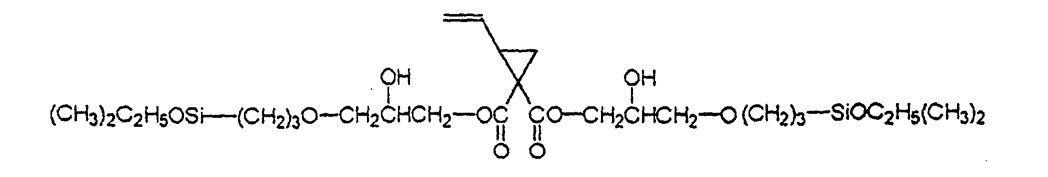

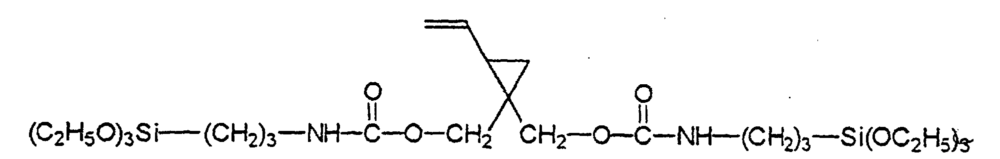

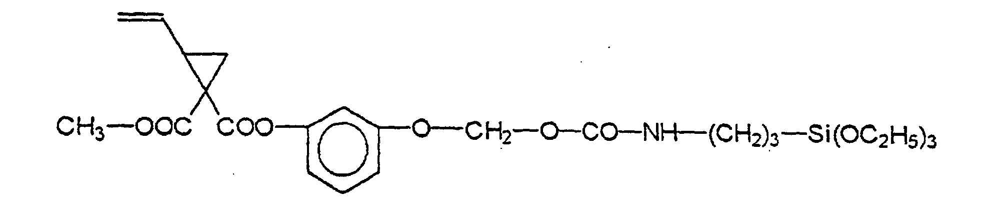

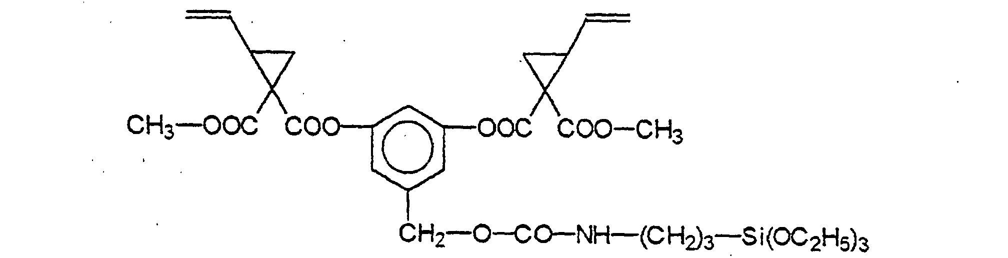

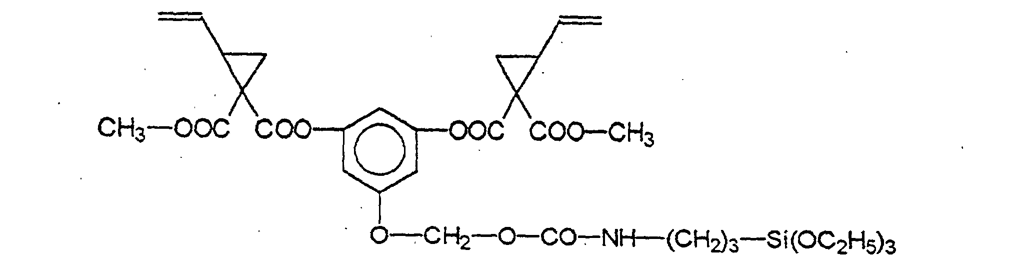

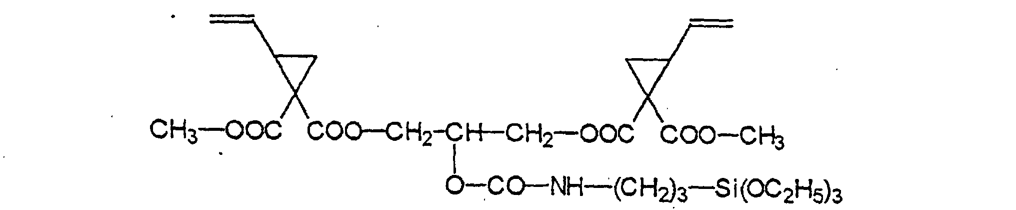

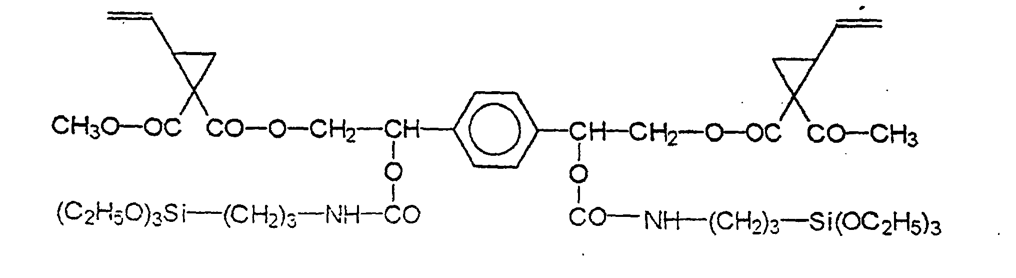

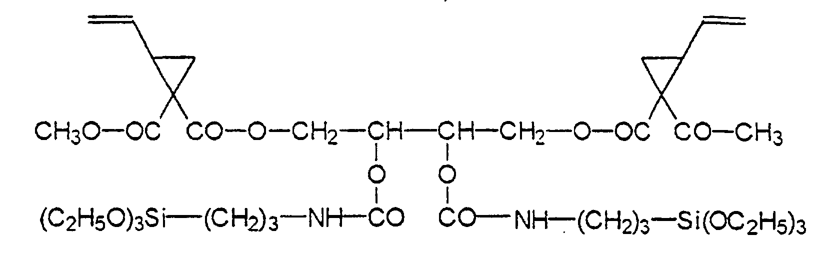

Die Herstellung der erfindungsgemäßen Vinylcyclopropansilane (I) ist insbesondere über eine große Anzahl von konventionellen Additions- oder Kondensationsreaktionen möglich, die nach den für diese Reaktionen üblichen Methoden durchgeführt werden. Verfahren die zur Herstellung der erfindungsgemäßen Silane eingesetzt werden können, sind z.B. in W. Noll , Chemie und Technologie der Silicone, 2. Auflage, Verlag Chemie, Weinheim, 1968, insbesondere S. 22 ff., sowie im Übersichtsartikel von R.C. Mehrotra in J. Non-Crystalline Solids 100, (1988) 1-15 und der in diesem Artikel zitierten Literatur beschrieben.

In einer ersten Variante kann z.B. 1-Methoxycarbonyl-2-vinylcyclopropan-1-carbonsäure (1) oder deren Säurechlorid oder Kaliumsalz mit Amino- oder Mercapto-funktionalisierten Silanen im Rahmen einer nucleophilen Substitution umgesetzt werden.

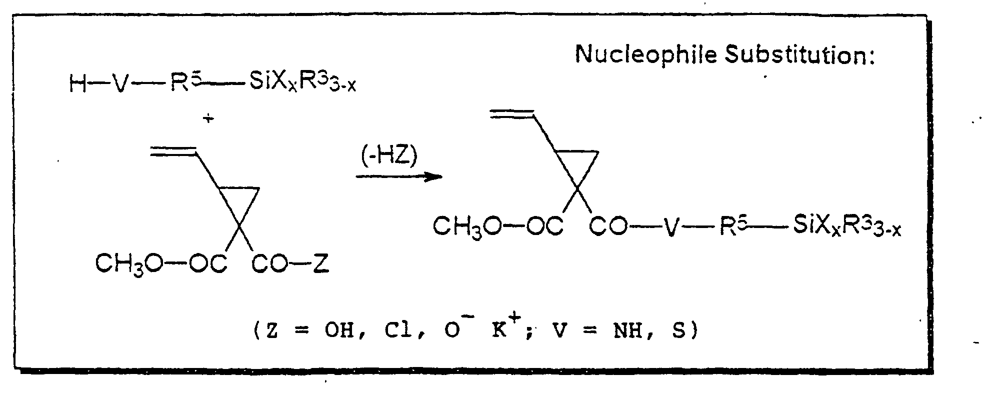

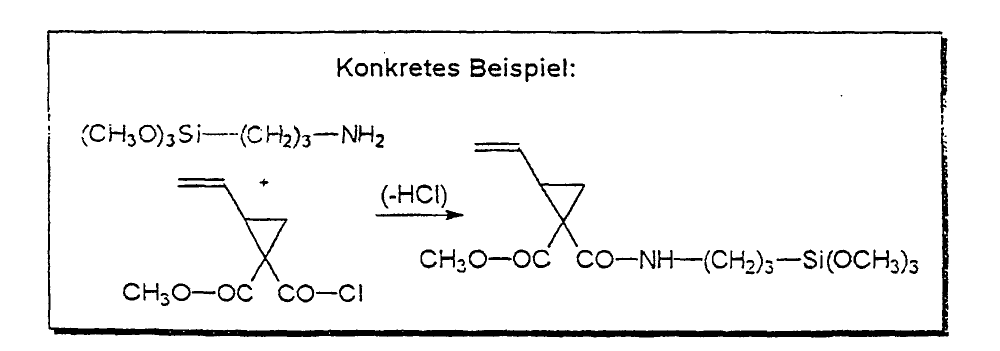

Analog dazu kann die Säure (1) auch an ein isocycanatgruppenhaltiges Silan addiert werden:

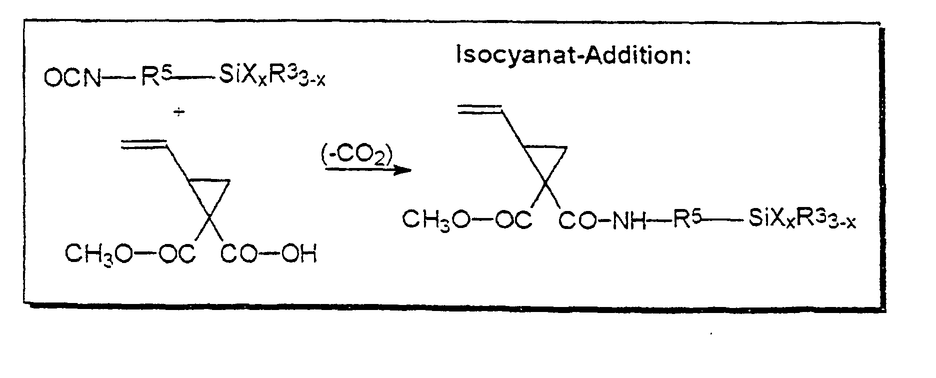

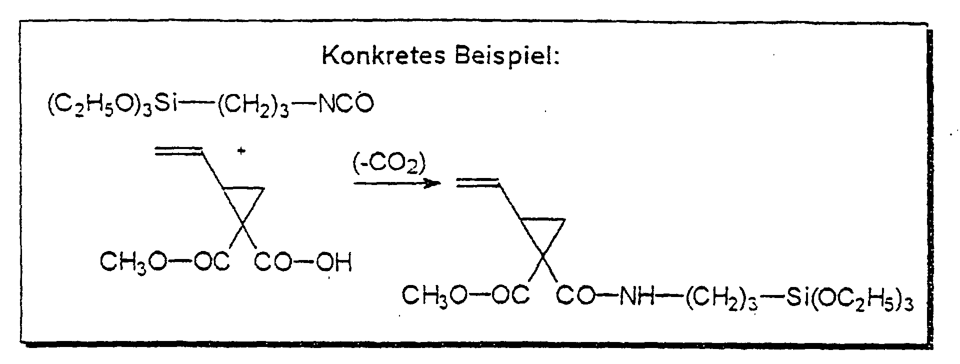

Ferner ist die Addition der Säure (1) an ein Epoxid-Silan möglich:

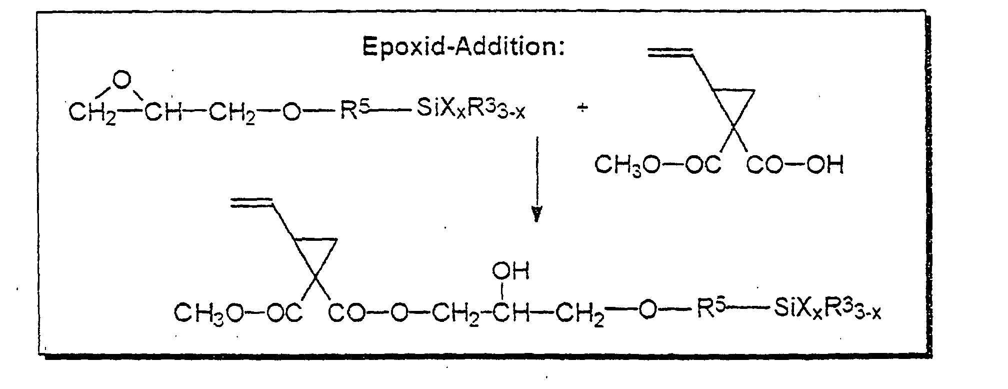

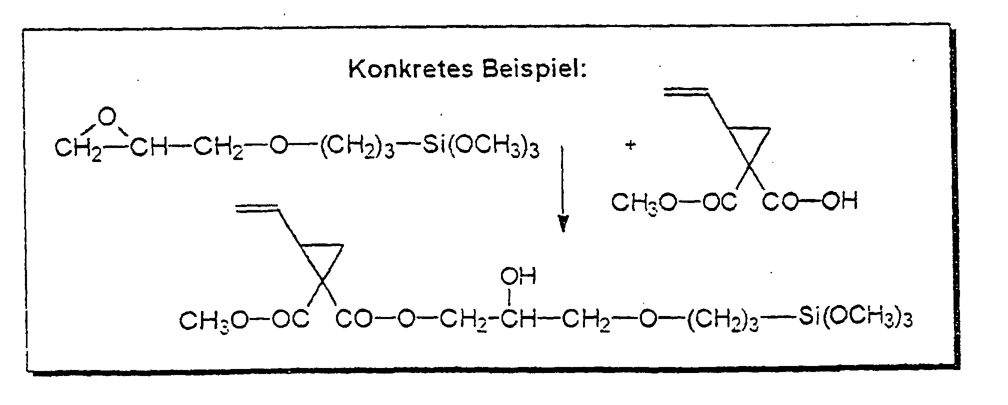

Entsprechend können auch Silane mit R = R³₃₋ₓXₓSi-R₄-R₁-R₂- hergestellt werden, wobei anstelle der Säure (1) die 2-Vinylcyclopropan-1,1-dicarbonsäure (2) oder deren Säurechlorid oder Kaliumsalz mit z.B. Amino- oder Mercapto-funktionalisierten Silanen im Rahmen einer nucleophilen Substitution umgesetzt wird.

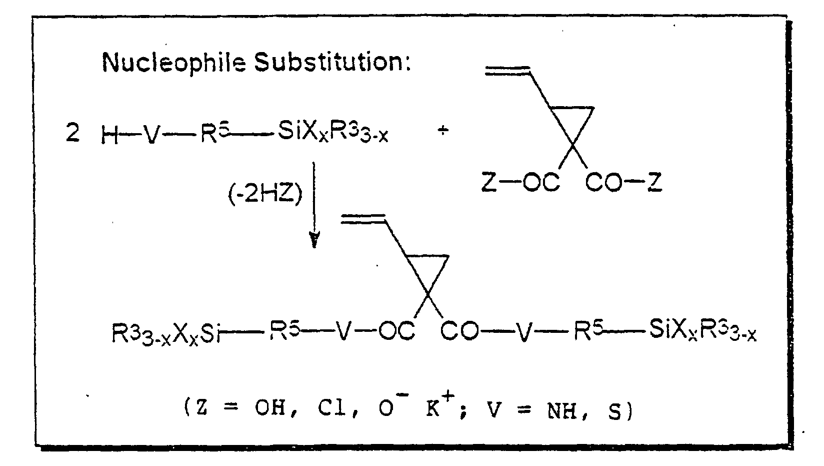

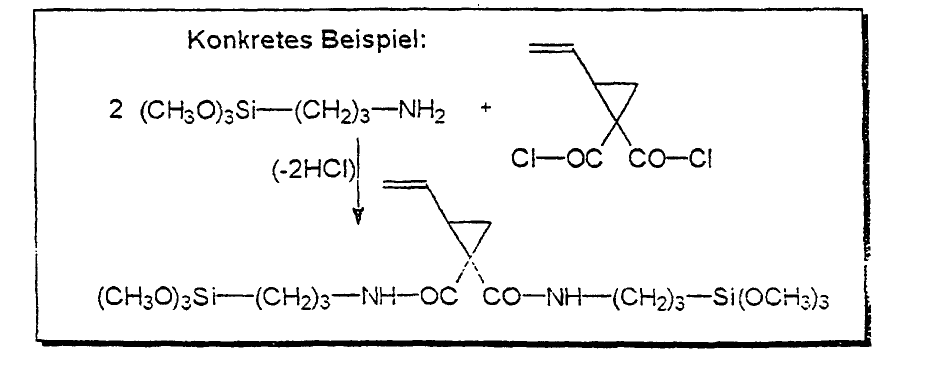

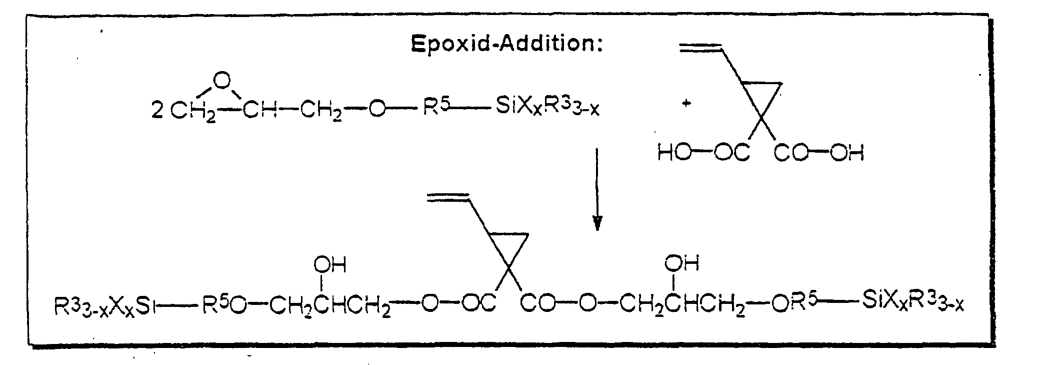

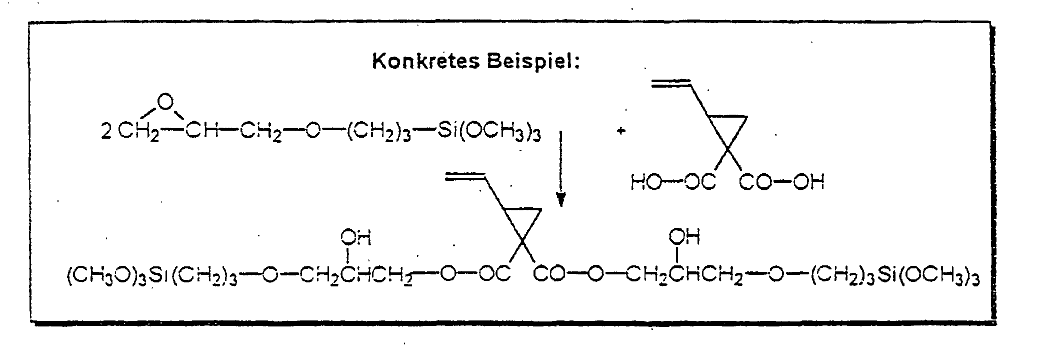

Schließlich läßt sich auch 1,1-Bis(hydroxymethyl)-2-vinylcyclopropan (3) an isocyanathaltige Silane addieren:

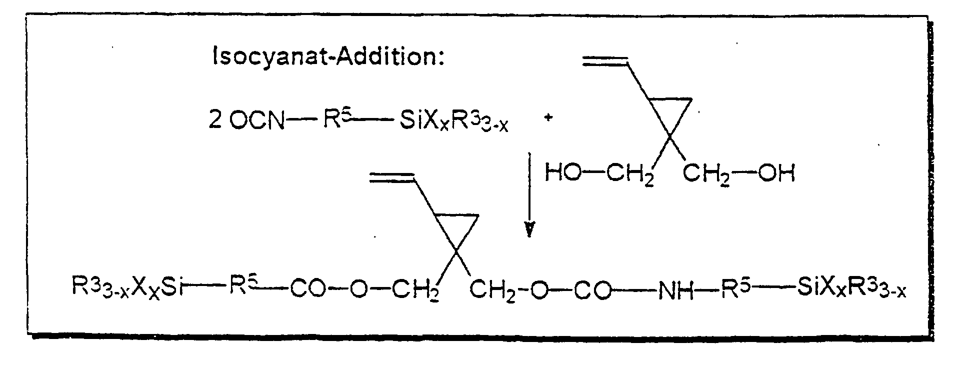

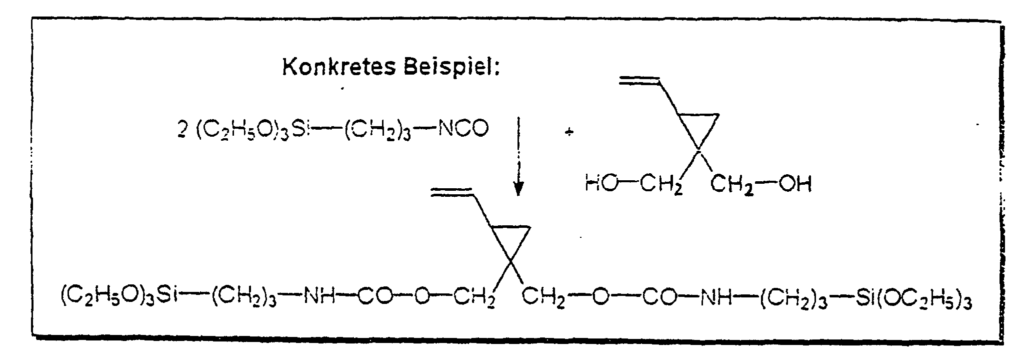

Die einzelnen Synthesemethoden können auch kombiniert werden. So sind z.B. multifunktionelle Silane durch Kombination der Epoxid-Addition mit der Isocyanat-Addition zugänglich, wie z.B.

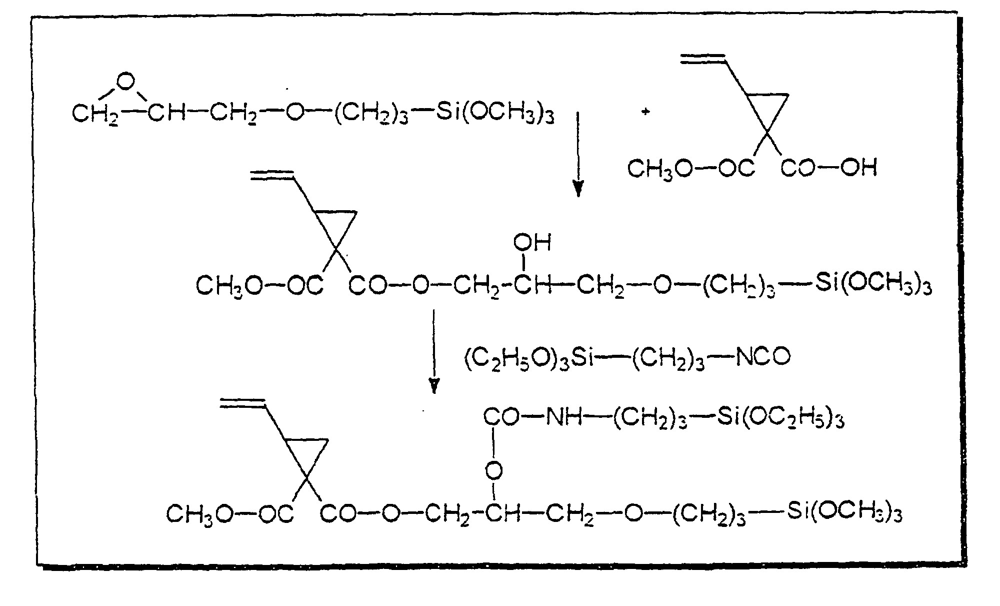

Die erfindungsgemäßen Silane (I) sind über die C=C-Doppelbindungen der 2-Vinylcyclopropanreste polymerisierbar und über die Reste X hydrolysierbar. Dabei führt die Polymerisation der 2-Vinylcyclopropangruppen zum Aufbau eines organischen Netzwerkes, während die hydrolysierbaren Gruppen durch Polykondensation ein anorganisches Polysiloxan-Netzwerk ergeben.

Die erfindungsgemäßen Vinylcyclopropansilane stellen Stoffe hoher Reaktivität dar, die bei Hydrolyse polymerisierbare Kieselsäurekondensate bilden, welche in Gegenwart von radikalischen Initiatoren thermisch oder durch Einstrahlung von Licht des sichtbaren oder UV-Bereichs zu mechanisch stabilen Schichten, Form- oder Füllkörpern polymerisiert werden können.

Durch geeignete Auswahl der bei der Herstellung der Vinylcyclopropansilane eingesetzten Edukte kann die Anzahl der hydrolysierbaren Gruppen, polymerisierbaren Gruppen und weiterer funktioneller Gruppen variiert werden. In Abhängigkeit von der Art und Anzahl der hydrolysierbaren Gruppen und der Anzahl der Vinylcylopropangruppen führt die Kondensation der Vinylcyclopropansilane und die Polymerisation der erhaltenen Kondensate zu Materialien mit Eigenschaften, die von silicongummiartig bis glasartig reichen.

Bei Anwesenheit von mindestens zwei Vinylcyclopropan-Resten ist die Ausbildung eines dreidimensionalen, organischen Netzwerkes möglich, wobei über den Abstand zwischen dem Si-Atom und dem Vinylcyclopropan-Rest, d.h. über die Länge der Spacergruppe, und durch Einbau weiterer funktioneller Gruppen die mechanischen Eigenschaften, wie z.B. Festigkeit und Flexibilität, und die physikalisch-chemischen Eigenschaften, z.B. Haftungsvermögen, Wasseraufnahme und Brechzahl, der erhaltenen Kieselsäurekondensate variiert und den Anforderungen des jeweiligen Anwendungsfalles optimal angepaßt werden können. Dabei führen aliphatische Gruppen zu eher flexiblen und aromatische Gruppen zu eher steifen Produkten.

Durch die Anzahl der polymerisierbaren Vinylcyclopropangruppen ist ferner die Vernetzungsdichte einstellbar, die dann ebenfalls die Eigenschaften und Einsatzmöglichkeiten der entsprechenden gebildeten Kieselsäurekondensate beeinflußt. Enthalten die erfindungsgemäßen Vinylcyclopropansilane darüber hinaus noch ionisch vernetzbare Gruppen als Substituenten, wie z.B. EpoxyGruppen, dann kann simultan oder konsekutiv, d.h. als 2-Stufen-Prozeß, durch deren ionische Polymerisation eine weitere Erhöhung der Vernetzungsdichte erreicht werden.

Die erfindungsgemäßen Vinylcyclopropansilane und deren Kieselsäurekondensate besitzen eine nur geringe Flüchtigkeit, so daß sie sich einfach und weitgehend unbedenklich verarbeiten lassen. Im Hinblick auf die vorstehend angegebenen Variationsmöglichkeiten der kondensierbaren und polymerisierbaren Reste der erfindungsgemäßen Vinylcyclopropansilane können daraus herstellbare Kieselsäurekondensate als Harze oder Füllstoffe für verschiedenste Anwendungsgebiete bereitgestellt werden können.

Die Silane (I) sind stabile Verbindungen, sie können entweder allein oder zusammen mit anderen hydrolysierbaren, kondensierbaren und/oder polymerisierbaren Komponenten zu den erfindungsgemäßen Kieselsäurekondensaten verarbeitet werden.

Neben den Silanen der Formel (I) können noch weitere hydrolytisch kondensierbare Verbindungen des Siliciums, Aluminiums, Titans, Zirkoniums oder Phosphors bei der Herstellung der erfindungsgemäßen Kieselsäurekondensate eingesetzt werden, die dann auch als Kieselsäure(hetero)kondensate bezeichnet werden. Diese Verbindungen können entweder als solche oder in bereits vorkondensierter Form verwendet werden. Bevorzugt ist es, wenn für die Herstellung der erfindungsgemäßen Kieselsäure(hetero)kondensate mindestens 20 Mol.%, besonders bevorzugt mindestens 80 Mol.%, auf der Basis monomerer Verbindungen, hydrolysierbare Siliciumverbindungen eingesetzt werden. Ebenso bevorzugt ist es, wenn zur Herstellung der Kieselsäure(hetero)kondensate mindestens 10 Mol.%, insbesondere 40 bis 100 Mol.%, jeweils auf der Basis monomerer Verbindungen, erfindungsgemäße Vinylcyclopropansilane verwendet werden.

Als weitere hydrolytisch kondensierbare Verbindungen wird bevorzugt mindestens ein Silan der allgemeinen Formel (VI) eingesetzt :

R¹⁰ ₖ(Z'R¹¹)ₘSiX'₄₋₍ₖ₊ₘ₎ (VI)

wobei R¹⁰, Z', R¹¹, X', k und m unabhängig voneinander die folgenden Bedeutungen haben:
- R¹⁰ =: C₁- bis C₈-Alkyl, C₂- bis C₁₂-Alkenyl oder C₆- bis C₁₄-Aryl;
- R¹¹ =: C₁- bis C₈-Alkylen, C₂- bis C₁₂-Alkenylen oder C₆- bis C₁₄-Arylen;
- X' =: Wasserstoff, Halogen oder C₁- bis C₈-Alkoxy;
- Z' =: Mercapto-, Glycidyl-, Acryl-, Methacryl-, Vinyl-, Allyl- oder Vinylethergruppe;
- k =: 0, 1, 2 oder 3;
- m =: 0, 1, 2 oder 3; und
- k+m =: 1, 2 oder 3.

Derartige Silane sind z.B. in der DE-C-34 07 087 beschrieben, und spezielle Beispiele für hydrolytisch kondensierbare Silane der allgemeinen Formel (VI) sind:

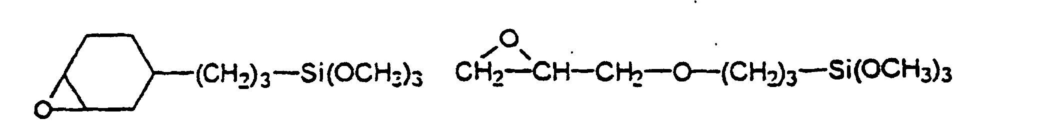

Darüber hinaus können als weitere bevorzugte hydrolytisch kondensierbare Verbindungen mindestens eine Zirkonium-, Titanoder Aluminium-Verbindung der Formeln

MeX"R¹² _{z} AlR¹³ ₃

eingesetzt werden, wobei Me, R¹², R¹³, X", y und z unabhängig voneinander die folgenden Bedeutungen haben:
- Me =: Zr oder Ti;
- R¹² =: Wasserstoff, substituiertes oder unsubstituiertes C₁-bis C₁₂-Alkyl, C₇- bis C₁₅-Alkylaryl oder C₆- bis C₁₄-Aryl;
- R¹³ =: Halogen, OH, C₁- bis C₈-Alkoxy;
- X" =: Halogen, OH, C₁- bis C₈-Alkoxy;
- y =: 1 bis 4, insbesondere 2 bis 4;
- z =: 1 bis 3, insbesondere 0 bis 2.

Bevorzugte Beispiele für einsetzbare Zirkonium- und TitanVerbindungen sind ZrCl₄, Zr(OC₂H₅)₄, Zr(OC₃H₇)₄, Zr(OC₄H₉)₄, ZrOCl₂, TiCl₄, Ti(OC₂H₅)₄, Ti(OC₃H₇)₄ und Ti(OC₄H₉)₄. Bevorzugte Beispiele für einsetzbare Aluminium-Verbindungen sind Al(OCH₃)₃, Al(OC₂H₅)₃, Al(OC₃H₇)₃, Al(OC₄H₉)₃ und AlCl₃.

Auch komplexierte Zr-, Ti- und Al-Verbindungen können eingesetzt werden, wobei als Komplexbildner u.a. Säuren oder β-Dicarbonylverbindungen fungieren können.

Weitere hydrolysierbare Verbindungen, die zur Herstellung der Kieselsäure(hetero)kondensate eingesetzt werden können, sind z.B. Bortrihalogenide, Zinntetrahalogenide, Zinntetraalkoxide und Vanadylverbindungen.

Die erfindungsgemäßen Kieselsäurekondensate der Silane (I) werden durch Hydrolyse der vorhandenen hydrolysierbaren Gruppen X, z.B. Alkoxy-Gruppen, und anschließende Kondensation erhalten, welche zur Ausbildung eines anorganischen Netzwerkes aus Si-O-Si-Einheiten führt. Die Hydrolyse und Kondensation erfolgt üblicherweise im basischen oder sauren Milieu, wobei eine Verknüpfung von C=C-Doppelbindungen, die in den eingesetzten Silanen enthalten sind, in der Regel unerwünscht ist.

Die erfindungsgemäßen Kieselsäurekondensate können auch in nicht vollständig hydrolysierter und kondensierter Form vorliegen. In solchen Fällen spricht man auch von sogenannten Vorkondensaten.

Bei der Herstellung der erfindungsgemäßen Kieselsäure(hetero)kondensate geht man üblicherweise so vor, daß man die gegebenenfalls in einem Lösungsmittel gelösten Silane (I) bei Raumtemperatur oder unter leichter Kühlung und in Gegenwart eines Hydrolyse- und Kondensationskatalysators mit der erforderlichen Menge Wasser versetzt und die entstehende Mischung ein bis mehrere Stunden lang rührt. Als Lösungsmittel kommen vor allem aliphatische Alkohole, wie z.B. Ethanol oder i-Propanol, Dialkylketone, wie Aceton oder Methylisobutylketon, Ether, wie z.B. Diethylether oder Tetrahydrofuran (THF), Ester, wie Ethyl- oder Butylacetat, und deren Mischungen in Frage.

Wird die hydrolytische Kondensation in Gegenwart reaktiver Zr-, Ti- oder Al-Verbindungen durchgeführt, so sollte die Wasserzugabe stufenweise bei ca. 0 bis 30 °C erfolgen. Dabei ist es meist günstig Wasser nicht als solches zuzugeben, sondern in Form von wasserhaltigen Lösungsmitteln, wie z.B. wäßrigem Ethanol, oder durch Freisetzung über eine chemische Reaktion, wie z.B. über eine Veresterung, zuzuführen.

Vorzugsweise erfolgt die Hydrolyse und Kondensation in Gegenwart eines Kondensationskatalysators, wobei protonen- oder hydroxylionen-abspaltende Verbindungen, wie organische oder anorganische Säuren oder Basen, bevorzugt sind. Besonders bevorzugt sind flüchtige Säuren oder Basen, insbesondere Salzsäure oder Ammoniak. Es hat sich bewährt, bei der Hydrolyse und Kondensation Verfahrensweisen der Sol-Gel-Technologie zu übernehmen, wie sie z.B. in C.J. Brinker et al., "Sol-Gel-Science", Academic Press, Boston, 1990, beschrieben sind. Das "Sol-Gel-Verfahren" ist darüber hinaus in DE-A-27 58 414, DE-A-27 58 415, DE-A-30 11 761, DE-A-38 26 715 und DE-A-38 35 968 offenbart.

Die erhaltenen Kieselsäure(hetero)kondensate der Silane (I) und gegebenenfalls weiterer hydrolytisch kondensierbare Verbindungen können entweder als solche oder nach teilweiser oder vollständiger Entfernung von verwendetem Lösungsmittel eingesetzt werden. In einigen Fällen kann es sich auch als vorteilhaft erweisen, das zur hydrolytischen Kondensation eingesetzte Lösungsmittel durch ein anderes Lösungsmittel zu ersetzen.

Die erfindungsgemäßen polymerisierbaren Kieselsäure(hetero)kondensate und die Silane (I) sowie Zusammensetzungen mit Gehalt an diesen Kondensaten oder Silanen können durch thermische, photochemische oder redox-induzierte Polymerisation gehärtet werden, wobei die Polymerisation üblicherweise nach Zugabe geeigneter Initiatoren und weiterer polymerisationsfähiger Komponenten erfolgt. Wenn unterschiedliche polymerisationsfähige Gruppen, z.B. Vinylcyclopropan- und Epoxid-Gruppen, vorhanden sind, können auch mehrere Härtungsmechnismen, z.B. radikalische und kationische Polymerisation, gleichzeitig oder in aufeinanderfolgenden Stufen benutzt werden.

Zur Initiierung der radikalischen Polymerisation werden vorzugsweise thermische Initiatoren und/oder Photoinitiatoren eingesetzt. Bevorzugte Beispiele für thermische Initiatoren sind die bekannten Peroxide, wie z.B. Dibenzoylperoxid, Dilaurylperoxid, tert.-Butylperoctoat oder tert.-Butylperbenzoat, und außerdem Azobisisobutyroethylester, Benzpinakol oder 2,2-Dimethylbenzpinakol.

Beispiele für geeignete Photoinitiatoren sind Benzophenon, Benzoin sowie deren Derivate oder α-Diketone oder deren Derivate, wie 9,10-Phenanthrenchinon, Diacetyl oder 4,4-Dichlorbenzil. Bevorzugt werden Campherchinon und 2,2-Methoxy-2-phenyl-acetophenon und besonders bevorzugt α-Diketone in Kombination mit Aminen als Reduktionsmittel, wie z.B. N-Cyanoethyl-N-methylanilin, 4-(N,N-Dimethylamino)benzoesäureester, N,N-Dimethylaminoethylmethacrylat, N,N-Dimethyl-sym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-N-propylphenylphosphinoxid, besonders geeignet.

Für die duale Aushärtung von radikalisch und kationisch polymerisierbaren Verbindungen eignen sich besonders Diaryliodonium- oder Triarylsulfoniumsalze, wie z.B. Triphenylsulfoniumhexafluorophosphat oder -hexafluorantimonat.

Als Initiatoren für eine bei Raumtemperatur durchgeführte Polymerisation werden Redox-Initiatorkombinationen, wie z.B. Kombinationen von Benzoyl- oder Laurylperoxid mit N,N-Dimethylsym.-xylidin oder N,N-Dimethyl-p-toluidin, verwendet.

In den erfindungsgemäßen Zusammensetzungen können neben den Silanen (I) oder den entsprechenden Kieselsäure(hetero)kondensaten auch geeignete polymerisationsfähige mono- oder multifunktionelle Monomere vorhanden sein, welche auch als Verdünnermonomere bezeichnet werden können. Besonders bevorzugte Verdünnermonomere sind dabei vor allem mono- und multifunktionelle Vinylcyclopropan-Derivate wie 1,1-Bis(alkoxycarbonyl)- oder 1,1-Bis(aryloxycarbonyl)-2-vinylcyclopropane, z.B. 1,1-Bis(methoxycarbonyl)-, 1,1-Bis(ethoxycarbonyl)- oder 1,1-Bis(phenoxycarbonyl)-2-vinylcyclopropan, oder Bis(2-vinyl-cyclopropan-1-alkoxycarbonyl-1-carbonyloxy)-Derivate, z.B. Bis(2-vinyl-cyclopropan-1-methoxycarbonyl-1-carbonyloxy)ethan oder Bis(2-vinyl-cyclopropan-1-methoxycarbonyl-1-carbonyloxy)benzol. Darüber hinaus sind auch andere radikalisch polymerisierbare Verdünnermonomere wie mono-funktionelle (Meth)acrylate, z.B. Methyl(meth)acrylat, Ethyl(meth)acrylat, Butyl(meth)acrylat, Benzyl(meth)acrylat, Furfuryl(meth)acrylat oder Phenyl(meth)acrylat, sowie mehrfunktionelle (Meth)acrylate, z.B. Bisphenyl-Adi(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- oder Tetraethylenglykoldi(meth)acrylat, Decandioldi(meth)acrylat, Trimethylolpropantri(meth)acrylat, Pentaerythrittetra(meth)acrylat, Butandioldi(meth)acrylat, 1,10-Decandioldi(meth)acrylat oder 1,12-Dodecandioldi(meth)acrylat, einsetzbar.

Die erfindungsgemäßen Silane, deren Kieselsäurekondensate oder Kieselsäureheterokondensate sowie diese enthaltende Zusammensetzungen können als solche oder in zumindest teilweise polymerisierter Form z.B. als Lacke zur Beschichtung von Kunststoffen, Glas oder anderen Substraten, als Füllstoffe oder Bulkmaterial für Komposite und besonders für medizinische Materialien, z.B. zur Herstellung von Kontaktlinsen, eingesetzt werden. Besonders bevorzugt werden sie jedoch als Dentalmaterial oder Bestandteil davon verwendet.

Gegebenenfalls können die erfindungsgemäßen Zusammensetzungen auch weitere Additive, wie z.B. Färbemittel (Pigmente oder Farbstoffe), Stabilisatoren, Aromastoffe, mikrobizide Wirkstoffe, Flammschutzmittel, Weichmacher oder UV-Absorber, enthalten.

Weitere mögliche Additive sind Füllstoffe. Beispiele für bevorzugte Füllstoffe sind Quarz-, Glaskeramik- oder Glaspulver, insbesondere Barium- oder Strontiumsilikatglas-Pulver, Lithium-Aluminium-Silikatglas-Pulver, Silicium-, Zirkonium- oder Aluminiumoxid, oder deren Mischungen, feinteilige Kieselsäuren, insbesondere pyrogene oder gefällte Kieselsäuren, und röntgenopake Füllstoffe, wie z.B. Ytterbiumtrifluorid.

Eine besonders bevorzugte erfindungsgemäße Zusammensetzung enthält:
(a) 5 bis 90, insbesondere 10 bis 70 Gew.-%, bezogen auf die Zusammensetzung, Kieselsäure(hetero)kondensat von einem Silan (I),
(b) 0 bis 80, insbesondere 0 bis 50 Gew.-%, bezogen auf die Zusammensetzung, Verdünnermonomer,
(c) 0,1 bis 5, insbesondere 0,2 bis 2,0 Gew.-%, bezogen auf die Zusammensetzung, Polymerisationsinitiator, und/oder
(d) 0 bis 90, insbesondere 0 bis 80 Gew.-%, bezogen auf die Zusammensetzung, Füllstoffe.

Besonders bevorzugt werden die erfindungsgemäßen Zusammensetzungen als dentaler Zement, dentales Füllungsmaterial oder dentales Bonding für Füllungsmaterialien eingesetzt. Die Verwendung der Zusammensetzungen erfolgt dabei insbesondere dadurch, daß sie auf den zu behandelnden Bereich eines künstlichen oder natürlichen Zahnes aufgebracht und durch Polymerisation gehärtet werden.

Dabei erweist es sich als besonderer Vorteil der erfindungsgemäßen Zusammensetzungen, daß sie einerseits einen geringen Polymerisationsschrumpf zeigen und andererseits zu Kompositmaterialien mit hoher mechanischer Festigkeit führen. Eine derartige Kombination von Eigenschaften ist gerade bei Dentalmaterialien von besonderer Bedeutung.

Die Erfindung wird nachfolgend anhand von Beispielen erläutert.

### Beispiel 1 :

### Synthese von 1-Methoxycarbonyl-1-[(3-triethoxysilyl)propylaminocarbonyl)]-2-vinylcyclopropan (4)

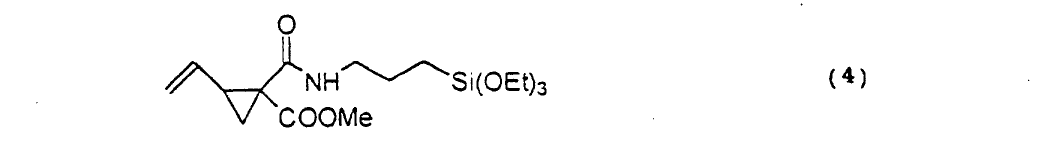

14,5 g (58,8 mmol) Isocyanatopropyltriethoxysilan wurden zu einer mit Eis gekühlten Lösung von 10 g (58,8 mmol) 2-Vinylcyclopropan-1,1-dicarbonsäuremonomethylester und 20 mg Dibutylzinndilaureat in 20 ml Methylenchlorid getropft. Nach 2 Tagen Rühren bei Raumtemperatur ließ sich mittels IR-Spektroskopie kein Isocyanat mehr nachweisen. Nach dem Abdestillieren blieben 22 g einer schwach gefärbten Flüssigkeit zurück.
- ¹H-NMR (CDCl₃) :: 0,6 (t, 2H, CH₂Si), 1,25 (m, 11H, CH₂, CH₃), 1,62 (m, 2H, CH₂), 2,6 (m, 1H, CH), 3,16 (t, 2H, CH₂), 3,8 (q, 9H, OCH₂, OCH₃), 4,80-5,00 (br, 1H, NH), 5,1-5,8 (m, 3H, CH=CH₂) ppm.
- IR (Film) :: 3355, 2979, 1718, 1081 cm⁻¹.

### Beispiel 2 :

### Synthese von 1,1-[Bis-(triethoxysilylpropylaminocarboxymethyl)]-2-vinylcyclopropan (5)

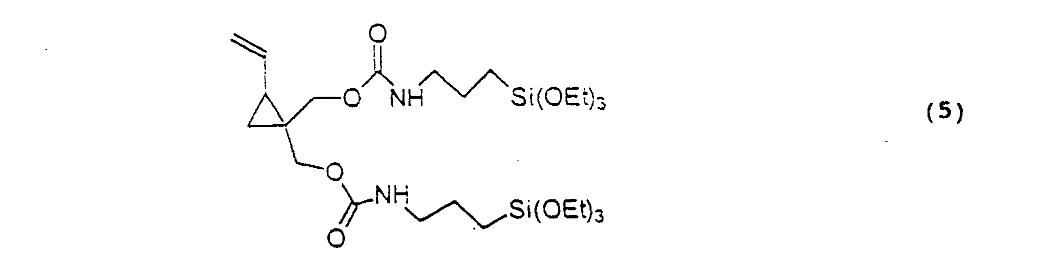

8 g (32 mmol) 3-Isocyanatopropyltriethoxysilan wurden vorsichtig zu einer Lösung von 2 g (16 mmol) Bis(hydroxymethyl)-2-vinylcyclopropan und 20 mg Dibutylzinndilaureat in 50 ml trockenem Methylenchlorid gegeben. Nach 48 h Rühren unter Rückfluß war mittels IR-Spektroskopie kein Isocyanat mehr nachweisbar. Nach dem Abdestillieren des Lösungsmittels verblieben 10 g (100 % Ausbeute) einer klaren Flüssigkeit.
- ¹H-NMR (CDCl₃) :: 5,00-5,65 (m, 3H, CH=CH₂), 4,99 (br, 2H, NH), 3,99 (m, 4H, CH₂O), 3,83 (q, 12H, CH₂O), 3,18 (t, 4H, CH₂N), 1,76 (m, 3H, CH-Cyclopropyl), 1,22 (m 22H, CH₂, CH₃), 0,61 (t, 4H, CH₂Si) ppm.
- IR (Film) :: 3444, 2976, 1718, 1517, 1266, 1077 cm⁻¹.

### Beispiel 3 :

### Synthese des Adduktes (6) von Isocyanatopropyltriethoxysilan an 1-Methoxycarbonyl-2-vinylcyclopropan-1-carbonsäure-(2-hydroxy-3-phenoxy)prop-1-yl-ester

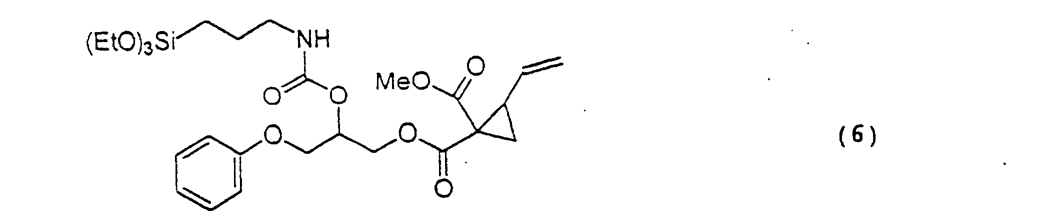

Unter Argon wurden 6,1 g (19 mmol) 1-Methoxycarbonyl-2-vinylcyclopropan-l-carbonsäure-(2-hydroxy-3-phenoxy)prop-1-yl-ester, welcher durch Addition von 1-Methoxycarbonyl-2-vinylcyclopropan-1-carbonsäure an Phenylglycidylether erhalten wurde, 4,7 g (19 mmol) Isocyanatopropyltriethoxysilan und 20 mg Dibutylzinndilaureat 4 Tage unter Rückfluß gerührt, bis mittels IR-Spektroskopie kein Isocyanat mehr nachweisbar war. Nach dem Abdestillieren des Lösungsmittels verblieben 10,5 g (97 % Ausbeute) einer gelben zähen Flüssigkeit.
- ¹H-NMR (CDCl₃) :: 0,5-0,6 (t, 2H, CH₂Si), 1,2 (m, 11H, CH₂, CH₃), 1,5-1,8 (m, 2H, CH₂), 2,6 (m, 1H, CH), 3,1-3,4 (t, 2H, CH₂), 3,6-3,9 (m, 11H, OCH₂, OCH₃), 4,1 (m, 1H, CHO), 5,1-5,4 (br, 1H, NH), 6,8-7,3 (5 H_{arom.}) ppm.
- IR (Film) :: 3385, 2974, 2928, 1729, 1600 cm⁻¹.

### Beispiel 4 :

### Di-Addukt (7) von 3-Isocyanatopropyltriethoxysilan an Bis[(((1,4-(2-vinyl-1-methoxycarbonyl)-cyclopropan-1-yl)-carbonyloxy)-2-hydroxypropoxy)-methyl]-cyclohexan

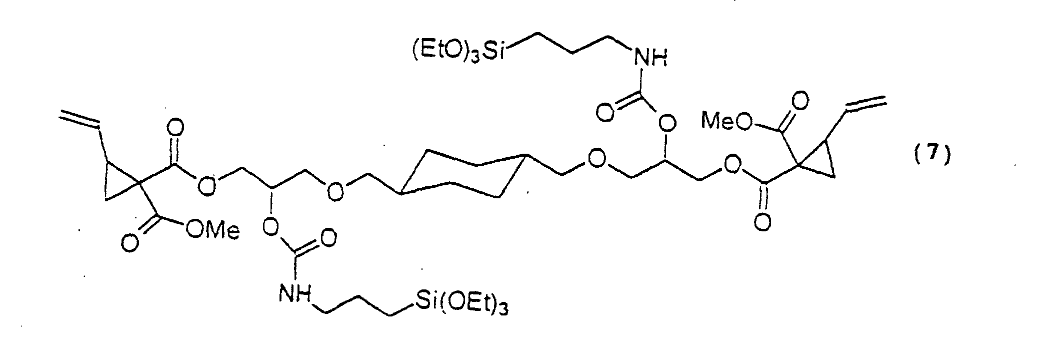

8 g (13,4 mmol) Bis[(((1,4-(2-vinyl-1-methoxycarbonyl)-cyclopropan-1-yl)-carbonyloxy)-2-hydroxypropoxy)-methyl]-cyclohexan (Di-Addukt von 1-Methoxycarbonyl-2-vinylcyclopropan-1-carbonsäure an 1,4-Cyclohexandimethanoldiglycidylether) und 6,6, g (26,8 mmol) 3-Isocyanatopropyltriethoxysilan und 20 mg Dibutylzinndilaureat wurden in 20 ml trockenem Methylenchlorid 4 Tage unter Rückfluß gekocht, bis mittels IR-Spektroskopie kein Isocyanat mehr nachgewiesen werden konnte. Nach Abdestillieren des Lösungsmittels verblieben 13,2 g (90 % Ausbeute) einer gelben, viskosen Flüssigkeit.
- ¹H-NMR (CDCl₃) :: 0,55-0,66 (t, 4H, CH₂Si), 1,15-1,25 (m, 22H, CH₃, -CH₂-CH₂-CH₂-), 1,3-1,8 (br. m, 14H, Cyclohexyl, Cyclopropyl-CH₂), 2,55-2,65 (m, 2H, CH), 3,1-3,3 (m, 4H, CH₂N), 3,4-3,7 (m, 12H, CH₂O), 3,75 (s, 6H, OCH₃), 3,85 (t, 4H, CH₂), 4,1-4,4 (m, 2H, CHO), 5,01 (br, 2H, NH), 5,25-5,55 (m, 6H, CH=CH₂), ppm.
- IR (Film) :: 3354, 2975, 1728, 1527, 1078 cm⁻¹.

### Beispiel 5 :

### Hydrolytische Kondensation von 1-Methoxycarbonyl-1-[(3-triethoxysilyl)propylaminocarbonyl)]-2-vinyl-cyclopropan (4)

100 mmol des Silans (**4**) wurden in 15 ml wasserfreiem Ethanol oder THF gelöst. Die Hydrolyse erfolgte durch Zugabe von 150 mmol Wasser in Form von 0,1 N wäßriger HCl. Nach 72 h Rühren bei Raumtemperatur wurden die flüchtigen Komponenten im Vakuum entfernt, und es entstand ein viskoses Harz, das als Monomerkomponente für eine radikalische Polymerisation, z.B. als Komponente für ein radikalisch härtbares Dentalmaterial, eingesetzt werden konnte.

### Beispiel 6 :

### Hydrolytische Kondensation von 1,1-[Bis-(triethoxysilylpropylaminocarboxymethyl)]-2-vinylcycloproapn (5) :

100 mmol des Silans (**5**) wurden in 25 ml wasserfreiem Ethanol oder THF gelöst. Die Hydrolyse erfolgte durch Zugabe von 150 mmol Wasser in Form von 0,1 N wäßriger HCl. Nach 2 bis 4 h Rühren bei Raumtemperatur wurden die flüchtigen Komponenten im Vakuum entfernt, und es entstand ein viskoses Harz, das nach Zugabe von radikalischem Initiator als Komponente für eine lichthärtende Beschichtung oder ein lichthärtendes Dentalmaterial eingesetzt werden konnte.

### Beispiel 7:

### Additionsprodukt von 3-Aminopropyltriethoxysilan an 1-(2-Acryloyloxyethyloxycarbonyl)-1-methoxycarbonyl-2-vinylcyclopropan

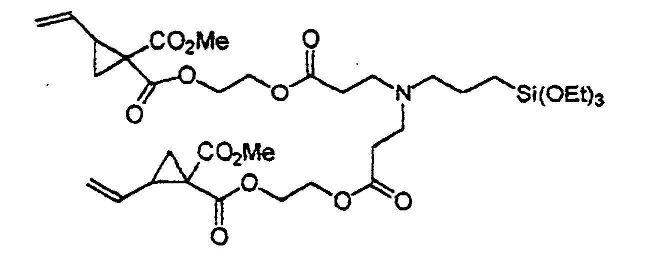

Eine Mischung von 6,2 g (28 mmol) 3-Aminopropyltriethoxysilan und 15,0 g (56 mmol) 1-(2-Acryloyloxyethyloxycarbonyl)-1-methoxycarbonyl-2-vinylcyclopropan in 20 ml Dichlormethan wurde bei Raumtemperatur gerührt. Nach 14 Tagen wurde das Lösungsmittel im Vakuum abdesilliert. Es verblieben 19,2 g (91 % Ausbeute) einer klaren viskosen Flüssigkeit.
- ¹H-NMR (CDCl₃) :: 0,56 (t, 2H, SiCH₂), 1,22 (t, 9H, CH₃), 1,50 (m, 2H, CH₂), 1,58-1,76 (m, 4H, Cyclopropyl-CH₂), 2,48 (m, 6H, NCH₂), 2,61 (m, 2H, Cyclopropyl-CH), 2,78 (t, 4H, OCCH₂), 3,74 (s, 6H, OCH₃), 3,82 (q, 6H, OCH₂), 4,28 (m, 8H, OCH₂) und 5,14-5,77 (m, 6H, CH=CH₂) ppm.
- IR (Film) :: 2974, 1737 und 1645 cm⁻¹.

### Beispiel 8:

### Additionsprodukt von 3-Mercaptopropyltriethoxysilan an 1-(2-Acryloyloxyethyloxycarbonyl)-1-methoxycarbonyl-2-vinylcyclopropan

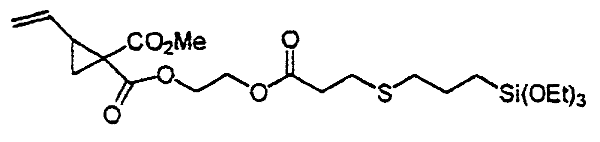

Eine Mischung von 29,0 g (0,1 mol) 1-(2-Acryloyloxyethyloxycarbonyl)-1-methoxycarbonyl-2-vinylcyclopropan, 24,5 g (0,1 mol) 3-Mercaptopropyltriethoxysilan und 70 mg 1,8-Diazabicyclo[5.4.0]undec-7-en (DBU) in 60 ml Acetonitril wurde 3 Tage bei Raumtemperatur gerührt. Nach Entfernung des Lösungsmittels im Vakuum verblieben 50 g (93 % Ausbeute) einer klaren, viskosen Flüssigkeit.
- ¹H-NMR (CDCl₃) :: 0,73 (t, 2H, SiCH₂), 1,24 (s, 12H, CH₃), 1,58-1,74 (m, 2H, Cyclopropyl-CH₂ und 2H, CH₂), 2,52-2,79 (m, 6H, SCH₂, OCH und Cyclopropyl-CH), 3,74 (s, 3H, OCH3), 3,82 (q, 6H, OCH₂), 4,32 (m, 4H, OCH₂) und 5,13-5,84 (m, 3H, CH=CH₂) ppm.
- IR Film:: 2974, 1738 und 1652 cm⁻¹.

### Beispiel 9:

### Synthese von 1-Methoxycarbonyl-2-vinylcyclopropan-1-carbonsäure-[2-hydroxy-3-(3-trimethoxysilylpropoxy)propyl]ester

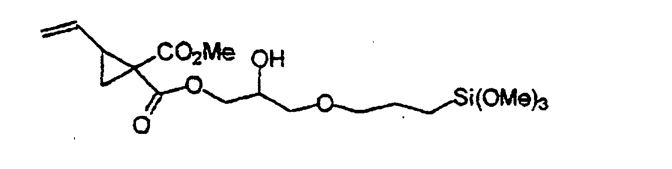

Eine Mischung von 10,0 g (58 mmol) 1-Methoxycarbonyl-2-vinylcyclopropancarbonsäure, 14,0 g Trimethoxysilylpropylglycidylether und 100 mg Lithiumperchlorat in 20 ml Dichloromethan wurden 15 Tage bei Raumtemperatur gerührt. Nach Entfernung des Lösungsmittels im Vakuum verblieben 20 g (83 % Ausbeute) einer klaren, gelben viskosen Flüssigkeit.
- ¹H-NMR (CDCl₃) :: 0,68 (br, 2H, SiCH₂), 1,73 (br, 2H, CH₂), 1,98-2,15 (m, 2H, Cyclopropyl-CH₂), 2,61 (m, 1H, Cyclopropyl-CH), 3,40-3,54 (m, 5H, OH-CH-CH₂OCH₂), 3,57 (s, 9H, OCH₃), 3,75 (d, 2H, OCH₂), 3,83 (s, 3H, OCH₃) und 5,24-5,63 (m, 3H, CH=CH₂) ppm.
- IR (Film) :: 3626, 2972, 1736 und 1645 cm⁻¹.

### Beispiel 10 :

### Synthese von 2-Vinylcyclopropan-1,1-dicarbonsäure-[N,N'-bis(3-triethoxysilylpropyl)]amid

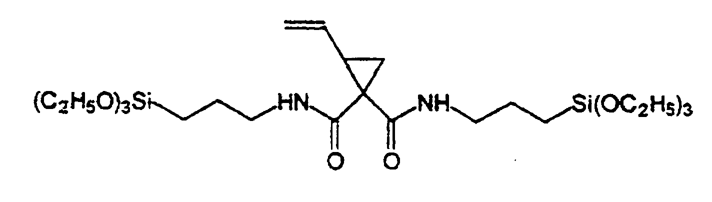

Eine Mischung von 10,0 g (65 mmol) 2-Vinylcyclopropan-1,1-dicarbonsäure, 31,7 g (130 mmol) 3-Isocyanatopropyltriethoxysilan und 40 mg Dibutylzinndilaurat in 100 ml wasserfreiem Aceton wurde 4 Tage bei Raumtemperatur gerührt. Nachdem mittels IR-Spektroskopie kein Isocyanat mehr nachweisbar war, wurde die Mischung filtriert und das Filtrat im Vakuum eingeengt. Es ergaben sich 26,5 g (72 % Ausbeute) eines dunkel gefärbten Oels.
- ¹H-NMR (CDCl₃) :: 0,62 (br, 4H, SiCH₂), 1,24 (t, 18H, CH₃), 1,60 (br, 4H, CH₂), 2,05-2,19 (m, 2H, Cyclopropyl-CH₂), 2,77 (m, 1H, Cyclopropyl-CH), 3,14 (t, 4H, NCH₂), 3,76 (s, 12H, OCH₂) und 5,22-5,89 (m, 3H, CH=CH₂) ppm.
- IR (Film) :: 3346, 2977, 1716 und 1362 cm⁻¹.

### Beispiel 11 :

### Hydrolytische Kondensation von 1-Methoxycarbonyl-1-[(3-triethoxysilyl)propylaminocarbonyl)]-2-vinylclopropan (4)

100 mmol des Silans (**4**) wurden in 35 ml wasserfreiem Tetrahydrofuran (THF) gelöst. Die Hydrolyse des Silans erfolgte durch Zugabe von 300 mmol Wasser in Form einer wäßrigen 0,1 N NH₄F-Lösung. Nach 22 h Rühren bei Raumtemperatur wurden die flüchtigen Komponenten im Vakuum entfernt. Das entstandene viskose Harz wurde in 30 ml THF gelöst und zur Silylierung noch vorhandener Si-OH Gruppen mit 100 mmol Collidin als Base und 100 mmol Trimethylchlorsilan (TMCS) unter Kühlung versetzt. Die Mischung wurde zur Vervollständigung der Reaktion ca. 12 h bei Raumtemperatur gerührt und dann wurde der entstandene Niederschlag abfiltriert. Vor dem Entfernen der flüchtigen Komponenten im Vakuum, wurden der Mischung 20 mmol Urethandimethacrylat UDMA (Ivoclar, Additionsprodukt von 2 Mol 2-Hydroxyethylmethacralyt an 1 Mol 2,2,4-Trimethylhexamethylendiisocyanat) als Verdünner zugesetzt. Nach dem Entfernen der flüchtigen Komponenten im Vakuum erhielt man ein viskoses Harz (η = 45 Pas (23° C)).

Nach Zugabe des Photoinitiators Lucirin TPO (BASF AG, 0,8 Gew.-%) wurden zur Untersuchung der Eigenschaften Prüfkörper entsprechend der ISO-Norm 4049 (1988) geformt und durch Bestrahlen mit einer dentalen Lichtquelle Spectramat (Vivadent) mit Licht einer Wellenlänge von 400-500 nm (2x3 Minuten) gehärtet. Dabei ergab sich ein Polymerisationsschrumpf von nur 4,5 Vol.-%. Anschließend wurden Biegefestigkeiten von 29 MPa bestimmt. Mittels Differential-Scanning-Kalorimetrie (DSC) konnte eine Glasübergangstemperatur T_{g} von 90° C ermittelt werden. Aufgrund dieser Eigenschaften konnte das Material für eine lichthärtende Beschichtung oder ein lichthärtendes Dentalmaterial eingesetzt werden.

### Beispiel 12 :

### Hydrolytische Kondensation von 1-Methoxycarbonyl-1-[(3-triethoxysilyl)propylaminocarbonyl)]-2-vinylcyclopropan (4) und Cokondensation mit Zr(OPr)₄

100 mmol des Silans (4) wurden in 15 ml wasserfreiem Ethanol gelöst. Die Vorhydrolyse des Silans erfolte durch Zugabe von 150 mmol Wasser in Form von einer wäßrigen 0,1 N NH₄F-Lösung. Nach 2 h Rühren bei Raumtemperatur erfolgte die Zugabe von 10 mmol des zuvor hergestellten Zr(OR)-Komplexes. Zur Herstellung des Zr(OR)-Komplexes wurden 100 mmol Zr(OPr)₄ (80 %ig in Propanol) unter Eiskühlung mit 100 mmol 2-Vinylcyclopropan-1, 1-dicarbonsäuremonoethylester versetzt und die Mischung wurde anschließend 2 h bei Raumtemperatur gerührt. Die Mischung aus Vorhydrolysat und Zr(OR)-Komplex wurde noch 6 h bei Raumtemperatur gerührt. Vor dem Entfernen der flüchtigen Komponenten im Vakuum wurden der Mischung 20 mmol 1,1,1-Tris-[(2-vinylcyclopropan-1-carbonsäuremethylester-1-carbonyloxy)methyl]propan sowie 30 mmol 2-Vinylcyclopropan-1,1-dicarbonsäuremethylester als Vernetzer und Verdünner zugesetzt. Nach dem Entfernen der flüchtigen Komponenten im Vakuum erhielt man ein viskoses Harz (η = 46 Pas (23° C)), das nach Zugabe eines radikalischen Photoinitiators analog Beispiel 11 als Komponente für eine lichthärtende Beschichtung oder ein lichthärtendes Dentalmaterial eingesetzt werden konnte.

### Beispiel 13 :

### Hydrolytische Kondensation von 1-Methoxycarbonyl-1-[(3-triethoxysilyl)propylaminocarbonyl)]-2-vinyl-cyclopropan (4) und Cokondensation mit Tetraethoxysilan

100 mmol Tetraethoxysilan (TEOS) wurden in 30 ml wasserfreiem Ethanol gelöst. Die Vorhydrolyse des TEOS erfolgte durch Zugabe von 150 mmol Wasser in Form von wäßriger 0,1 N Salzsäure. Nach 15 Minuten Rühren bei Raumtemperatur wurden 25 mmol des Silans (4) zu dem Vorhydrolysat gegeben und es wurde mit 37,5 mmol Wasser in Form von 0,1 N Salzsäure hydrolysiert. Nach 72 h Rühren bei Raumtemperatur wurden die flüchtigen Komponenten im Vakuum entfernt, und es entstand ein viskoses Harz, das nach Zugabe eines radikalischen Photoinitiators analog Beispiel 11 als Komponente für eine lichthärtende Beschichtung oder ein lichthärtendes Dentalmaterial eingesetzt werden konnte.

### Beispiel 14:

### Hydrolytische Kondensation des Adduktes (6) aus Beispiel 3

100 mmol des Silans (**6**) wurden in 55 ml wasserfreiem THF gelöst. Die Hydrolyse des Silans erfolgte durch Zugabe von 300 mmol Wasser in Form von wäßriger 0,1 N Salzsäure. Nach 25 h Rühren bei Raumtemperatur wurden die flüchtigen Komponenten im Vakuum entfernt. Das entstandene viskose Harz wurde in 50 ml THF gelöst und zur Silylierung noch vorhandener Si-OH Gruppen mit 100 mmol Collidin als Base und 100 mmol TMCS unter Kühlung versetzt. Die Mischung wurde zur Vervollständigung der Reaktion ca. 12 h bei Raumtemperatur gerührt und dann wurde der entstandene Niederschlag an Collidinhydrochlorid abfiltriert. Vor dem Entfernen der flüchtigen Komponenten im Vakuum, wurden der Mischung 30 mmol Urethandimethacrylat UDMA als Verdünner zugesetzt. Nach dem Entfernen der flüchtigen Komponenten im Vakuum erhielt man ein viskoses Harz (η = 65 Pas (23° C)). Nach Zugabe des Photoinitiators Lucirin TPO (0,8 Gew.-%) wurde das Harz analog Beispiel 11 ausgehärtet, wobei sich ein Polymerisationsschrumpf von nur 3,7 Vol.-% und ein T_{g} von 68° C ergab.

### Beispiel 15 :

### Hydrolytische Kondensation des Adduktes (6) aus Beispiel 3 und Cokondensation mit Zr(OPr)₄

100 mmol des Silans (6) wurden in 25 ml wasserfreiem Ethanol gelöst. Die Vorhydrolyse des Silans erfolgte durch Zugabe von 150 mmol Wasser in Form einer wäßrigen 0,1 N NH₄F-Lösung. Nach 2 h Rühren bei Raumtemperatur erfolgte die Zugabe von 10 mmol des zuvor hergestellten Zr(OR)-Komplexes. Zur Herstellung des Zr(OR)-Komplexes wurden 100 mmol Zr(OPr)₄ (80 %-ig in Propanol) unter Eiskühlung mit 100 mmol 2-Vinylcyclopropan-1,1-dicarbonsäuremonoethylester versetzt und anschließend wurde 2 h bei Raumtemperatur gerührt. Die Mischung aus Vorhydrolysat und Zr-Komplex wurde noch 15 h bei Raumtemperatur gerührt. Vor dem Entfernen der flüchtigen Komponenten im Vakuum, wurden der Mischung 20 mmol 1,1,1-Tris-[(2-vinylcyclopropan-1-carbonsäuremethylester-1-carbonyloxy)methyl]propan sowie 30 mmol 2-Vinylcyclopropan-1,1-dicarbonsäuremethylester als Vernetzer und Verdünner zugesetzt. Nach dem Entfernen der flüchtigen Komponenten im Vakuum erhielt man ein viskoses Harz (η = 120 Pas (23° C)), das nach Zugabe eines radikalischen Photoinitiators analog Beispiel 11 als Komponente für eine lichthärtende Beschichtung oder ein lichthärtendes Dentalmaterial eingesetzt werden konnte.

### Beispiel 16 :

### Hydrolytische Kondensation des Adduktes (6) aus Beispiel 3 und Cokondensation mit Tetraethoxysilan

100 mmol TEOS wurden in 35 ml wasserfreiem Ethanol gelöst. Die Vorhydrolyse des TEOS erfolgte durch Zugabe von 150 mmol Wasser in Form von 0,1 N Salzsäure. Nach 15 Minuten Rühren bei Raumtemperatur wurden 25 mmol des Silans (**6**) zu dem Vorhydrolysat gegeben und es wurde mit 37,5 mmol Wasser in Form von 0,1 N Salzsäure hydrolysiert. Nach 72 h Rühren bei Raumtemperatur wurden die flüchtigen Komponenten im Vakuum entfernt, und es entstand ein viskoses und nach Zugabe von Photoinitiator lichthärtendes Harz, welches als Komponente eines Dentalmaterials verwendet werden konnte.

### Beispiel 17 :

### Hydrolytische Kondensation des Di-Adduktes (7) aus Beispiel 4 und Cokondensation mit Diphenyldimethoxysilan

100 mmol des Silans (**7**) und 100 mmol Diphenyldimethoxysilan (DPhDMS) wurden in 30 ml wasserfreiem THF gelöst. Die Hydrolyse der Silane erfolgte durch Zugabe von 500 mmol Wasser in Form von 0,1 N Salzsäure. Nach 36 h Rühren bei Raumtemperatur wurden die flüchtigen Komponenten im Vakuum entfernt und es entstand ein viskoses Harz, das nach Zugabe eines radikalischen Initiators als Komponente für ein lichthärtendes Dentalmaterial eingesetzt werden konnte.

### Beispiel 18:

### Hydrolytische Kondensation des Silans aus Beispiel 7

100 mmol des Silans aus Beispiel 7 wurden in 75 ml wasserfreiem THF gelöst. Die Hydrolyse des Silans erfolgte durch Zugabe von 300 mmol Wasser in Form einer wäßrigen 0,1 N NH₄F-Lösung. Nach 22 h Rühren bei Raumtemperatur wurden die flüchtigen Komponenten im Vakuum entfernt. Das entstandene viskose Harz wurde in 60 ml THF gelöst und zur Silylierung noch vorhandener Si-OH Gruppen mit 100 mmol Collidin als Base und 100 mmol TMCS unter Kühlung versetzt. Die Mischung wurde zur Vervollständigung der Reaktion ca. 12 h bei Raumtemperatur gerührt und dann wurde der entstandene Niederschlag an Collidinhydrochlorid abfiltriert wird. Vor dem Entfernen der flüchtigen Komponenten im Vakuum, wurden der Mischung 30 mmol Urethandimethacrylat UDMA als Verdünner zugesetzt. Nach dem Entfernen der flüchtigen Komponenten im Vakuum erhielt man ein viskoses Harz (η = 80 Pas (23° C)). Nach Zugabe des Photoinitiator Lucirin TPO (0,8 Gew.-%) wurde das Harz analog Beispiel 11 ausgehärtet, wobei sich eine Biegefestigkeit von 40,5 MPa, ein Polymerisationsschrumpf von nur 3,9 Vol.-% und ein T_{g} von 76° C ergab.

### Beispiel 19 :

### Hydrolytische Kondensation des Silans aus Beispiel 7 und Cokondensation mit Tetraethoxysilan

25 mmol TEOS wurden in 45 ml wasserfreiem Ethanol gelöst. Die Vorhydrolyse des TEOS erfolgte durch Zugabe von 37,5 mmol Wasser in Form von 0,1 N Salzsäure. Nach 15 Minuten Rühren bei Raumtemperatur wurden 100 mmol des Silans aus Beispiel 7 zu dem Vorhydrolysat gegeben und es wurde mit 150 mmol Wasser in Form von 0,1 N Salzsäure hydrolysiert. Nach 72 h Rühren bei Raumtemperatur wurden die flüchtigen Komponenten im Vakuum entfernt, und es entstand ein viskoses Harz, das nach Zugabe eines radikalischen Initiators als Komponente für eine lichthärtende Beschichtung oder ein lichthärtendes Dentalmaterial eingesetzt werden konnte.

### Beispiel 20 :

### Hydrolytische Kondensation des Silans aus Beispiel 8

100 mmol des Silans aus Beispiel 8 wurden in 30 ml wasserfreiem THF gelöst. Die Hydrolyse des Silans erfolgte durch Zugabe von 300 mmol Wasser in Form einer wäßrigen 0,1 N NH₄F-Lösung. Nach 36 h Rühren bei Raumtemperatur wurden die flüchtigen Komponenten im Vakuum entfernt und es entstand ein viskoses Harz, das nach Zugabe eines radikalischen Initiators als Komponente für ein lichthärtendes Dentalmaterial eingesetzt werden konnte.

### Beispiel 21 :

### Hydrolytische Kondensation des Silans aus Beispiel 8 und Cokondensation mit Al(OBu)₃

100 mmol des Silans aus Beispiel 8 wurden in 25 ml wasserfreiem THF gelöst. Die Vorhydrolyse des Silans erfolgte durch Zugabe von 150 mmol Wasser in Form einer wäßrigen 0,1 N NH₄F-Lösung. Nach 20 h Rühren bei Raumtemperatur erfolgte die Zugabe von 10 mmol des zuvor hergestellten Al(OR)-Komplexes. Zur Herstellung des Al(OR)-Komplexes wurden 100 mmol Al(OBu)₃ in 55 ml THF gelöst und unter Eiskühlung mit 200 mmol 2-Vinylcyclopropan-1,1-dicarbonsäuremonoethylester versetzt und anschließend wurde 2 h bei Raumtemperatur gerührt. Nach 72 h Rühren bei Raumtemperatur wurden die flüchtigen Komponenten im Vakuum entfernt und es entstand ein viskoses Harz, das nach Zugabe eines radikalischen Initiators als Komponente für ein lichthärtendes Dentalmaterial eingesetzt werden konnte.

## Patentansprüche

1. Hydrolysierbare und polymerisierbare Vinylcyclopropansilane der allgemeinen Formel (I) und Stereoisomere davon 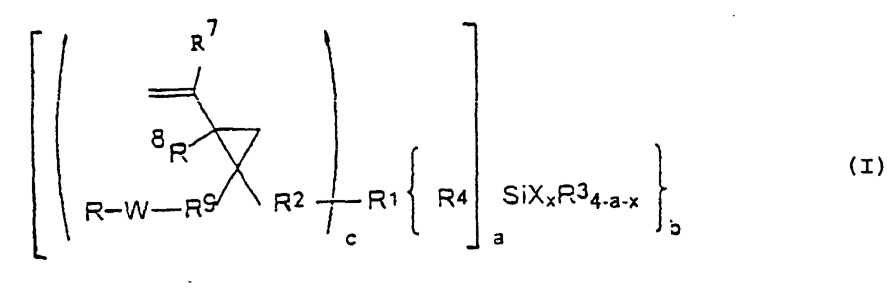 wobei die Variablen R, R¹, R², R³, R⁴, R⁵, R⁶, R⁷, R⁸, R⁹, W, X, Y, a, b, c und x unabhängig voneinander die folgenden Bedeutungen haben:
R = Wasserstoff, substituiertes oder unsubstituiertes C₁- bis C₁₂-Alkyl, C₇- bis C₁₅-Alkylaryl oder C₆- bis C₁₄-Aryl oder R³₃₋ₓXₓSi-R₄-R₁-R₂-;
R¹ = entfällt oder substituiertes oder unsubstituiertes C₁- bis C₁₈-Alkylen, C₆- bis C₁₈-Arylen, C₇- bis C₁₈-Alkylenarylen oder -Arylenalkylen, wobei diese Reste durch mindestens eine Gruppe ausgewählt aus Ether-, Thioether-, Ester-, Carbonyl-, Amid- und Urethangruppe unterbrochen sein oder diese endständig tragen können;
R² = entfällt oder substituiertes oder unsubstituiertes C₁- bis C₁₈-Alkylen, C₆- bis C₁₈-Arylen, C₇- bis C₁₈-Alkylenarylen oder C₇- bis C₁₈- Arylenalkylen, wobei diese Reste durch mindestens eine Gruppe ausgewählt aus Ether-, Thioether-, Ester-, Thioester-, Carbonyl-, Amid- und Urethangruppe unterbrochen sein oder diese endständig tragen können;
R³ = entfällt oder substituiertes oder unsubstituiertes C₁- bis C₁₈-Alkyl, C₂- bis C₁₈-Alkenyl, C₆- bis C₁₈-Aryl, C₇- bis C₁₈-Alkylaryl oder C₇- bis C₁₈-Arylalkyl, wobei diese Reste durch mindestens eine Gruppe ausgewählt aus Ether-, Thioether-, Ester-, Carbonyl-, Amid- und Urethangruppe unterbrochen sein können;
R⁴ = entfällt oder substituiertes oder unsubstituiertes -CHR⁶-CHR⁶-, -CHR⁶-CHR⁶-S-R⁵, -S-R⁵-, -Y-CO-NH-R⁵- oder -CO-O-R⁵-;
R⁵ = substituiertes oder unsubstituiertes C₁- bis C₁₈-Alkylen, C₆- bis C₁₈-Arylen, C₆- bis C₁₈-Alkylenarylen oder C₆- bis C₁₈-Arylenalkylen, wobei diese Reste durch mindestens eine Gruppe ausgewählt aus Ether-, Thioether-, Ester-, Carbonyl-, Amid- und Urethangruppe unterbrochen sein können;
R⁶ = Wasserstoff, oder substituiertes oder unsubstituiertes C₁- bis C₁₈-Alkyl oder C₆ bis C₁₀-Aryl;
R⁷ = Wasserstoff, oder substituiertes oder unsubstituiertes C₁- bis C₁₀-Alkyl, oder Halogen oder Hydroxy;
R⁸ = Wasserstoff oder substituiertes oder unsubstituiertes C₁- bis C₁₀-Alkyl;
R⁹ = entfällt oder substituiertes oder unsubstituiertes C₁- bis C₁₀-Alkylen;
W = entfällt oder Carbonyl-, Ester-, Ether-, Thioether-, Amid- oder Urethangruppe;
X = eine hydrolysierbare Gruppe, nämlich Halogen, Hydroxy, Alkoxy oder Acyloxy;
Y = O oder S;
a = 1, 2 oder 3;
b = 1, 2 oder 3;
c = 1 bis 6; und
x = 1, 2 oder 3;
und mit der Maßgabe, daß
(i) a+x = 2, 3 oder 4
und
(ii) a und/oder b = 1.

2. Vinylcyclopropansilane nach Anspruch 1, **dadurch gekennzeichnet, daß** mindestens eine der Variablen der Formel (I) unabhängig von den übrigen Variablen, die folgende Bedeutung hat:
R = C₁- bis C₅-Alkyl, Benzyl oder Phenyl oder R³₃₋ₓXₓSi-R₄-R₁-R₂-;
R¹ = C₁- bis C₈-Alkylen, wobei diese Reste durch mindestens eine Gruppe ausgewählt aus Ether-, Thioether-, Ester- und Urethangruppe unterbrochen sein können;
R² = entfällt oder C₁- bis C₈-Alkylen, wobei diese Reste durch mindestens eine Gruppe ausgewählt aus Ether-, Thioether-, Ester-, Thioester, Carbonyl-, Amid- und Urethangruppe unterbrochen sein oder diese endständig tragen können;
R³ = entfällt oder Methyl, Ethyl oder Phenyl;
R⁴ = entfällt oder -CHR⁶-CHR⁶-, -S-R⁵-, -Y-CO-NH-R⁵- oder -CO-O-R⁵-;
R⁵ = C₁- bis C₈-Alkylen, wobei diese Reste durch mindestens eine Gruppe ausgewählt aus Ether-, Thioether-, Ester-, Carbonyl-, Amid- und Urethangruppe unterbrochen sein können;
R⁶ = Wasserstoff oder C₁- bis C₅-Alkyl;
R⁷ = Wasserstoff oder C₁- bis C₅-Alkyl;
R⁸ = Wasserstoff oder C₁- bis C₅-Alkyl;
R⁹ = entfällt oder C₁- bis C₃-Alkylen;
W = Ester-, Amid- oder Urethangruppe;
X = Methoxy, Ethoxy oder Chlor;
Y = O oder S;
a = 1;
b = 1;
c = 1 bis 6;
x = 2 oder 3; und/oder
a+x = 3 oder 4.

3. Vinylcyclopropansilane nach Anspruch 1 oder 2, **dadurch gekennzeichnet, daß** in der Formel (I) unabhängig von den übrigen Variablen, mindestens eine der Variablen a, b und c = 1.

4. Vinylcyclopropansilan der Formel:

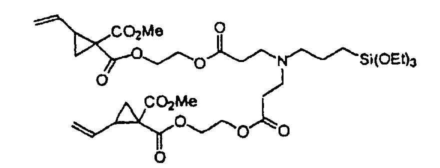

5. Polymerisierbare Kieselsäurekondensate der Vinylcyclopropansilane gemäß einem der Ansprüche 1 bis 4, welche durch Hydrolyse und Kondensation der Vinylcyclopropansilane (I), ggf. in Gegenwart weiterer hydrolysierbarer Verbindungen, oder des Vinylcyclopropylsilans gemäß Anspruch 4, ggf. in Gegenwart von Tetraethoxysilan erhältlich sind.

6. Polymerisate der Vinylcyclopropansilane gemäß einem der Ansprüche 1 bis 4 oder der Kieselsäurekondensate gemäß Anspruch 5.

7. Zusammensetzungen mit Gehalt an den Vinylcyclopropansilanen gemäß einem der Ansprüche 1 bis 4 oder an den Kieselsäurekondensaten gemäß Anspruch 5.

8. Zusammensetzungen nach Anspruch 7, die
(a) 5 bis 90, insbesondere 10 bis 70 Gew.-%, bezogen auf die Zusammensetzung, Kieselsäurekondensat von einem Silan (I) gemäß Anspruch 5,
(b) 0 bis 80, insbesondere 0 bis 50 Gew.-%, bezogen auf die Zusammensetzung, Verdünnermonomer,
(c) 0,1 bis 5, insbesondere 0,2 bis 2,0 Gew.-%, bezogen auf die Zusammensetzung, Polymerisationsinitiator, und
(d) 0 bis 90, insbesondere 0 bis 80 Gew.-%, bezogen auf die Zusammensetzung, Füllstoffe
enthalten.

9. Verwendung von den Vinylcyclopropansilanen gemäß einem der Ansprüche 1 bis 4, den Kieselsäurekondensaten nach Anspruch 5, den Polymerisaten nach Anspruch 6 oder den Zusammensetzungen nach Anspruch 7 oder 8 als Dentalmaterial oder als Bestandteil von Dentalmaterial.

## Claims

1. Hydrolysable and polymerizable vinylcyclopropane silanes of the general formula (I) and stereoisomers thereof 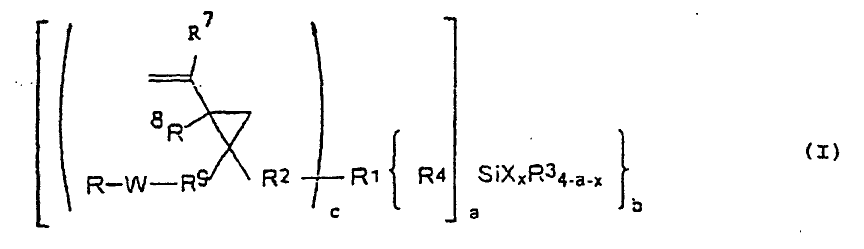 in which the variables R, R¹, R², R³, R⁴, R⁵, R⁶, R⁷, R⁸, R⁹, W, X, Y, a, b, c and x independently of one another have the following meanings:
R = hydrogen, substituted or unsubstituted C₁ to C₁₂ alkyl, C₇ to C₁₅ alkylaryl or C₆ to C₁₄ aryl or R³₃₋ₓXₓ Si-R₄-R₁-R₂-;
R¹ = absent or substituted or unsubstituted C₁ to C₁₈ alkylene, C₆ to C₁₈ arylene, C₇ to C₁₈ alkylenearylene or C₇ to C₁₈ arylenealkylene, where these radicals can be interrupted by or terminated with at least one group selected from the ether, thioether, ester, carbonyl, amide and urethane group;
R² = absent or substituted or unsubstituted C₁ to C₁₈ alkylene, C₆ to C₁₈ arylene, C₇ to C₁₈ alkylenearylene or C₇ to C₁₈ arylenealkylene, where these radicals can be interrupted by or terminated with at least one group selected from the ether, thioether, ester, thioester, carbonyl, amide and urethane group;
R³ = absent or substituted or unsubstituted C₁ to C₁₈ alkyl, C₂ to C₁₈ alkenyl, C₆ to C₁₈ aryl, C₇ to C₁₈ alkylaryl or C₇ to C₁₈ arylalkyl, where these radicals can be interrupted by at least one group selected from the ether, thioether, ester, carbonyl, amide and urethane group;
R⁴ = absent or substituted or unsubstituted -CHR⁶-CHR⁶-, -CHR⁶-CHR⁶-S-R⁵, -S-R⁵-, -Y-CO-NH-R⁵- or -CO-O-R⁵ -;
R⁵ = substituted or unsubstituted C₁ to C₁₈ alkylene, C₆ to C₁₈ arylene, C₆ to C₁₈ alkylenearylene or C₆ to C₁₈ arylenealkylene, where these radicals can be interrupted by at least one group selected from the ether, thioether, ester, carbonyl, amide and urethane group;
R⁶ = hydrogen, or substituted or unsubstituted C₁ to C₁₈ alkyl or C₆ to C₁₀ aryl;
R⁷ = hydrogen, or substituted or unsubstituted C₁ to C₁₀ alkyl or halogen or hydroxy;
R⁸ = hydrogen or substituted or unsubstituted C₁ to C₁₀ alkyl;
R⁹ = absent or substituted or unsubstituted C₁ to C₁₀ alkylene;
W = absent or carbonyl, ester, ether, thioether, amide or urethane group;
X = a hydrolysable group, namely halogen, hydroxy, alkoxy or acyloxy;
Y = O or S;
a = 1, 2 or 3;
b = 1, 2 or 3;
c = 1 to 6; and
x = 1, 2 or 3;
and with the proviso that
(i) a+x = 2, 3 or 4
and
(ii) a and/or b = 1.

2. Vinylcyclopropane silanes according to Claim 1, **characterized in that** at least one of the variables of formula (I), independently of the other variables, has the following meaning:
R = C₁ to C₅ alkyl, benzyl or phenyl or R³₃₋ₓXₓSi-R₄-R₁-R₂- ;
R¹ = C₁ to C₈ alkylene, where these radicals can be interrupted by at least one group selected from the ether, thioether, ester and urethane group;
R² = absent or C₁ to C₈ alkylene, where these radicals can be interrupted by or terminated with at least one group selected from the ether, thioether, ester, thioester, carbonyl, amide and urethane group;
R³ = absent or methyl, ethyl or phenyl;
R⁴ = absent or -CHR⁶-CHR⁶-, -S-R⁵-, -Y-CO-NH-R⁵- or -CO-O-_{R}⁵-;
R⁵ = C₁ to C₈ alkylene, where these radicals can be interrupted by at least one group selected from the ether, thioether, ester, carbonyl, amide and urethane group;
R⁶ = hydrogen or C₁ to C₅ alkyl;
R⁷ = hydrogen or C₁ to C₅ alkyl;
R⁸ = hydrogen or C₁ to C₅ alkyl;
R⁹ = absent or C₁ to C₃ alkylene;
W = ester, amide or urethane group;
X = methoxy, ethoxy or chloro;
Y = O or S;
a = 1;
b = 1;
c = 1 to 6;
x = 2 or 3; and/or
a+x = 3 or 4.

3. Vinylcyclopropane silanes according to Claim 1, **characterized in that** in formula (I), independently of the other variables, at least one of the variables a, b and c is 1.

4. Vinylcyclopropane silane of the formula:

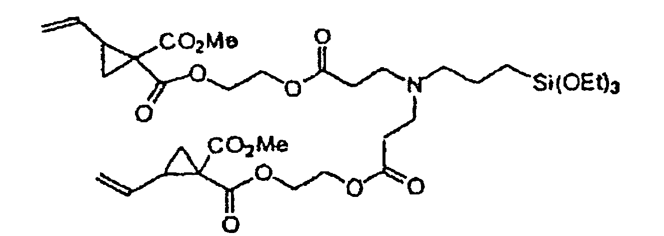

5. Polymerizable silica condensates of the vinylcyclopropane silanes according to any of Claims 1 to 4, which are obtainable via hydrolysis and condensation of vinylcyclopropane silanes (I), where appropriate in the presence of other hydrolysable compounds, or of the vinylcyclopropyl silane according to Claim 4, where appropriate in the presence of tetraethoxysilane.

6. Polymers of the vinylcyclopropane silanes according to any of Claims 1 to 4 or of the silica condensates according to Claim 5.

7. Compositions with content of the vinylcyclopropane silanes according to any of Claims 1 to 4 or of the silica condensates according to Claim 5.

8. Compositions according to Claim 7, which comprise
(a) from 5 to 90% by weight, in particular from 10 to 70% by weight, based on the composition, of silica condensate of a silane (I) according to Claim 5,
(b) from 0 to 80% by weight, in particular from 0 to 50% by weight, based on the composition, of diluent monomer,
(c) from 0.1 to 5% by weight, in particular from 0.2 to 2.0% by weight, based on the composition, of polymerization initiator, and
(d) from 0 to 90% by weight, in particular from 0 to 80% by weight, based on the composition, of fillers.

9. The use of the vinylcyclopropane silanes according to any of Claims 1 to 4, of the silica condensates according to Claim 5, of the polymers according to Claim 6 or of the compositions according to Claim 7 or 8 as a dental material or as a constituent of dental material.

## Revendications

1. Vinylcyclopropanesilanes hydrolysables et polymérisables répondant à la formule générale (I) et stéréoisomères de ces derniers 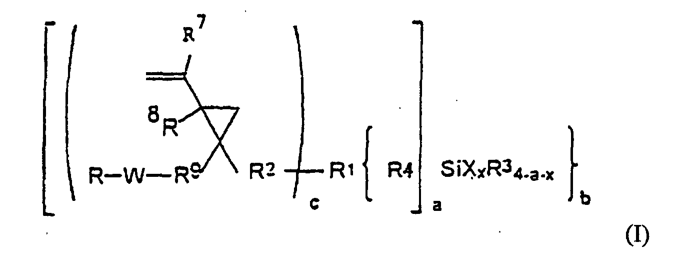 dans laquelle les variables R, R¹, R², R³, R⁴, R⁵, R⁶, R⁷, R⁸, R⁹, W, X, Y, a, b, c et x ont indépendamment les unes des autres les significations suivantes:
R = Hydrogène, alkyle en C₁ à C₁₂, alkylaryle en C₁ à C₁₅, ou aryle en C₆ à C₁₄, substitués ou non substitués ou R³₃₋ₓXₓSi-R⁴-R¹-R²-;
R¹ = absent ou alkylène en C₁ à C₁₈, arylène en C₆ à C₁₈, alkylènarylène en C₇ à C₁₈, ou arylènalkylène en C₇ à C₁₈, substitués ou non substitués, ces restes pouvant être interrompus par au moins un groupe choisi parmi les groupes éther, thioéther, ester, carbonyle, amide et uréthanne ou porter ceux-ci en extrémité;
R² = absent ou alkylène en C₁ à C₁₈, arylène en C₆ à C₁₈, alkylènarylène en C₇ à C₁₈, ou arylènalkylène en C₇ à C₁₈, substitués ou non substitués, ces restes pouvant être interrompus par au moins un groupe choisi parmi les groupes éther, thioéther, ester, thioester, carbonyle, amide et uréthanne ou porter ces derniers en extrémité;
R³ = absent ou alkyle en C₁ à C₁₈, alcényle en C₂ à C₁₈, aryle en C₆ à C₁₈, alkylaryle en C₇ à C₁₈, ou arylalkyle en C₇ à C₁₈, ces restes pouvant être interrompus par au moins un groupe choisi parmi les groupes éther, thioéther, ester, carbonyle, amide et uréthanne;
R⁴ = absent ou -CHR⁶ -CHR⁶ -, -CHR⁶ -CHR⁶ -S-R⁵, -S-R⁵-, Y-CO-NH- R⁵ - ou -CO-O-R⁵- substitués ou non substitués;
R⁵ = alkylène en C₁ à C₁₈, arylène en C₆ à C₁₈, alkylènarylène en C₆ à C₁₈, ou arylènalkylène en C₆ à C₁₈, substitués ou non substitués, ces restes pouvant être interrompus par au moins un groupe choisi parmi les groupes éther, thioéther, ester, carbonyle, amide et uréthanne;
R⁶ = hydrogène ou alkyle en C₁ à C₁₈ ou aryle en C₆ à C₁₀, substitués ou non substitués;
R⁷ = hydrogène ou alkyle en C₁ à C₁₀ substitué ou non substitué, ou halogène ou hydroxy;
R⁸ = hydrogène ou alkyle en C₁ à C₁₀ substitué ou non substitué;
R⁹ = absent ou alkylène en C₁ à C₁₀ substitué ou non substitué;
W = absent ou groupe carbonyle, ester, éther, thioéther, amide ou uréthanne;
X = un groupe hydrolysable, à savoir halogène, hydroxy, alcoxy, ou acyloxy;
Y = 0 ou S;
a = 1, 2 ou 3;
b = 1, 2 ou 3;
c = 1 à 6; et
x = 1, 2 ou 3;
et à la condition que
(i) a + x = 2, 3 ou 4
et
(il) a et/ou b = 1.

2. Vinylcyclopropanesilanes selon la revendication 1, **caractérisés en ce qu'**au moins l'une des variables de la formule (I) ait, indépendamment des autres variables, la signification suivante:
R = alkyle en C₁ à C₅, benzyle ou phényle ou R³₃₋ₓXₓSi-R⁴-R¹-R²-;
R¹ = alkylène en C₁ à C₈, ces restes pouvant être interrompus par au moins un groupe choisi parmi les groupes éther, thioéther, ester, et uréthanne ;
R² = absent ou alkylène en C₁ à C₈, ces restes pouvant être interrompus par au moins un groupe choisi parmi les groupes éther, thioéther, ester, thioester, carbonyle, amide et uréthanne ou porter ceux-ci en extrémité;
R³ = absent ou méthyle, éthyle ou phényle.
R⁴ = absent ou -CHR⁶ -CHR⁶ -, -S-R⁵-, Y-CO-NH- R⁵ - ou -CO-O-R⁵-;
R⁵ = alkylène en C₁ à C₈, ces restes pouvant être interrompus par au moins un groupe choisi parmi les groupes éther, thioéther, ester, carbonyle, amide et uréthanne;
R⁶ = hydrogène ou alkyle en C₁ à C₅;
R⁷ = hydrogène ou alkyle en C₁ à C₅;
R⁸ = hydrogène ou alkyle en C₁ à C₅;
R⁹ = absent ou alkylène en C₁ à C₃;
W =groupe ester, amide ou uréthanne;
X = méthoxy, éthoxy ou chlore;
Y = 0 ou S;
a = 1, ;
b = 1;
c = 1 à 6; et
x = 2 ou 3; et/ou
a+x = 3 ou 4

3. Vinylcyclopropanesilanes selon la revendication 1 ou 2, **caractérisés en ce que**, dans la formule (I), indépendamment des autres variables, au moins une des variables a, b et c = 1.

4. Vinylcyclopropanesilane répondant à la formule:

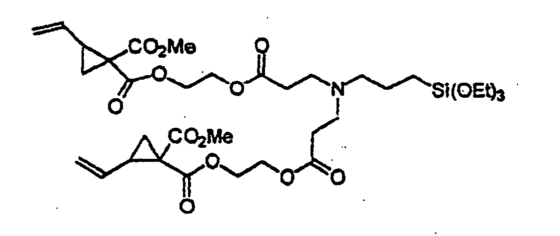

5. Condensats siliciques polymérisables des vinylcyclopropanesilanes selon l'une quelconque des revendications 1 à 4, susceptibles d'être obtenus par hydrolyse et condensation des Vinylcyclopropanesilanes (I), le cas échéant en présence d'autres composés hydrolysables ou du vinylcyclopropylsilane selon la revendication 4, le cas échéant en présence de tétraéthoxysilane.

6. Polymérisats des vinylcyclopropanesilanes selon l'une quelconque des revendications 1 à 4 ou des condensats siliciques selon la revendication 5.

7. Compositions présentant une teneur en les vinylcyclopropanesilanes selon l'une quelconque des revendications 1 à 4 ou en les condensats siliciques selon la revendication 5.

8. Compositions selon la revendication 7, qui contiennent
(a) 5 à 90, en particulier 10 à 70 % en poids, par rapport à la composition, de condensat silicique d'un silane (I) selon la revendication 5,
(b) 0 à 80, en particulier de 0 à 50 % en poids, par rapport à la composition, de monomère de dilution,
(c) 0,1 à 5, en particulier 0,2 à 2,0 % en poids, par rapport à la composition, d'initiateur de polymérisation, et
(d) 0 à 90, en particulier 0 à 80 % en poids, par rapport à la composition, de charges.

9. Utilisation des vinylcyclopropanesilanes selon l'une quelconque des revendications 1 à 4, des condensats siliciques selon la revendication 5, des polymérisats selon la revendication 6 ou des compositions selon les revendications 7 ou 8, comme matériau dentaire ou comme constituant de matériau dentaire.
